(19) Europäisches Patentamt
European Patent Office
Office européen des brevets

(11) **EP 1 109 819 B1**

(12) **EUROPÄISCHE PATENTSCHRIFT**

(45) Veröffentlichungstag und Bekanntmachung des Hinweises auf die Patenterteilung:
**16.04.2003 Patentblatt 2003/16**

(21) Anmeldenummer: **99944471.4**

(22) Anmeldetag: **21.08.1999**

(51) Int Cl.7: **C07F 17/02**, B01J 31/22, B01J 31/18, C07C 67/303, C07C 209/10, C07C 213/08

(86) Internationale Anmeldenummer:
**PCT/EP99/06153**

(87) Internationale Veröffentlichungsnummer:
**WO 00/014096 (16.03.2000 Gazette 2000/11)**

(54) **CHIRALE DIPHOSPHONITE AUF FERROCENBASIS FÜR DIE ASYMMETRISCHE KATALYSE**

FERROCENE-BASED DIPHOSPHONITES FOR ASYMMETRICAL CATALYSIS

DIPHOSPHONITES CHIRALES A BASE DE FERROCENE POUR LA CATALYSE ASYMETRIQUE

(84) Benannte Vertragsstaaten:
**AT BE CH CY DE DK ES FI FR GB GR IE IT LI LU MC NL PT SE**

(30) Priorität: **04.09.1998 DE 19840279**

(43) Veröffentlichungstag der Anmeldung:
**27.06.2001 Patentblatt 2001/26**

(73) Patentinhaber: **Studiengesellschaft Kohle mbH**
**45470 Mülheim an der Ruhr (DE)**

(72) Erfinder:
- **REETZ, Manfred, T.**
  **D-45470 Mülheim an der Ruhr (DE)**
- **GOSBERG, Andreas**
  **D-45470 Mülheim an der Ruhr (DE)**

(74) Vertreter: **von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

(56) Entgegenhaltungen:
**US-A- 5 171 892** **US-A- 5 817 850**

- **ERIC OOSTEROM, G. ET AL: "Catalysis in the core of a carbosilane dendrimer" CHEM. COMMUN. (CAMBRIDGE) (1999), (12), 1119-1120 , XP002121419**
- **REETZ, MANFRED T. ET AL: "Diphosphonites as highly efficient ligands for enantioselective rhodium-catalyzed hydrogenation" CHEM. COMMUN. (CAMBRIDGE) (1998), (19), 2077-2078 , XP002121420**
- **NIFANT'EV, I. E. ET AL: "Ferrocenylene diphosphonites as ligands in the synthesis of oligonuclear heterometallic complexes" ZH. OBSHCH. KHIM. (1995), 65(5), 756-60 , XP002121421**
- **BURK, MARK J. ET AL: "New chiral 1,1'-bis(phospholano)ferrocene ligands for asymmetric catalysi" TETRAHEDRON LETT. (1994), 35(50), 9363-6 , XP002121422**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung beinhaltet neue chirale 1,1'-Ferrocenylendiphosphonite und deren Synthese sowie Komplexe dieser Verbindungen mit Metallen der Gruppen VIIb, VIIIb und Ib des Periodensystems sowie deren Verwendung zur enantioselektiven Hydrierung von Olefinen, Ketonen und Iminen.

Die katalytische enantioselektive Synthese hat in den letzten 20 Jahren industriell an Bedeutung gewonnen, so z.B. die Übergangsmetall-katalysierte asymmetrische Hydrierung (B. Cornils, W. A. Herrmann, *Applied Homogeneous Catalysis with Organometallic Compounds*, Wiley-VCH, Weinheim, **1996;** R. Noyori, *Asymmetric Catalysis in Organic Synthesis*, Wiley, New York, **1994**). Als Katalysatoren werden gewöhnlich Rhodium-, Ruthenium- oder Iridium-Komplexe von optisch aktiven Diphosphanen wie BINAP (R. Noyori *et al., J. Am. Chem. Soc.* **1980,** *102*, 7932), DuPHOS (M. J. Burk *et al., J. Am. Chem. Soc.* **1995,** *117,* 9375), BICP (X. Zhang *et al., J. Am. Chem. Soc.* **1997,** *119*, 1799) und BPE (M. J. Burk *et al., J. Am. Chem. Soc.,* **1996,** *118*, 5142) verwendet. Nachteilig bei diesen Systemen ist der relative hohe präparative Aufwand bei der Darstellung und, ggf., der Antipodentrennung der racemischen Liganden sowie die oftmals unzureichende Enantioselektivität, die bei der Katalyse beobachtet wird. Es ist daher das Ziel der industriellen und akademischen Forschung, neue und besonders leistungsfähige Liganden auf möglichst einfachem Weg herzustellen.

Im Gegensatz zu Diphosphanen wurden chirale Diphosphonite als Liganden in der Katalyse nur in zwei Fällen beschrieben (L. Dahlenburg *et al., J. Organomet. Chem.* **1998,** *564,* 227 sowie *Eur. J. Inorg. Chem.* **1998,** *1*, 885 und I. E. Nifant'ev *et al.*, *Russ. J. Gen. Chem.* **1995,** *65*, 682.).

Verwendet wurden im ersten Fall Diphosphonite, die sich von enantiomerenreinem 1,2-Bis(dichlorphosphino) cyclopentan und achiralen einwertigen Alkoholen oder enantiomerenreinem (*R*)-Binaphthol ableiten. Mit solchen Liganden ließen sich in der Rhodium-katalysierten Hydrierung von 2-Acetamidozimtsäure Enantiomerenüberschüsse von max. 78 % im Falle des entsprechenden von Phenol abgeleiteten Diphosphonits erzielen. Die verwendeten Substrat/Katalysator Verhältnisse waren in allen Fällen extrem niedrig (76:1). Zudem wird auf erhebliche präparative Schwierigkeiten bei der Herstellung der Rhodium-Komplexe dieser Liganden hingewiesen. Beides sind gravierende Nachteile, die einer praktischen Verwendbarkeit entgegenstehen.

Nifant'ev *et al.* verwendeten Ferrocenylendiphosphonite auf der Basis von geschützten Monosacchariden, genauer $C_1$-symmetrischen aliphatischen 1,2-Diolen (2 Beispiele) und 1,3-Diolen sowie $C_2$-symmetrischen aliphatischen 1,4-Diolen (je 1 Beispiel). Rhodium-Komplexe dieser Liganden wurden ausgehend von $[Rh(CO)_2Cl]_2$ als Vorstufe synthetisiert und in der asymmetrischen Hydrosilylierung von Acetophenon eingesetzt. Die höchsten dabei erreichten *ee*-Werte betrugen 32 % bei zudem unzureichender Chemoselektivität, so daß eine kommerzielle Verwendbarkeit auszuschließen ist.

Ferrocenylendiphosphonite stellen jedoch, unseren Ergebnissen zufolge, bei Wahl geeigneter chiraler Diole als Ausgangsverbindungen, Liganden mit ausgezeichneten Eigenschaften dar. Zudem sind sie sehr leicht und kostengünstig herzustellen. Es zeigte sich dabei, daß als Diole in erster Linie $C_2$-symmetrische aliphatische 1,2-Diole oder axial chirale aromatische bzw. heteroaromatische Diole in Frage kommen. Wesentlich für die erfolgreiche Anwendung von Diphosphoniten ist also nicht nur die Wahl des geeigneten Rückgrates (backbone), im vorliegenden Fall Ferrocen, sondern auch die Wahl geeigneter Diole. Die beiden bisher in der Literatur bekannten Beispiele (s.o.) haben dies unberücksichtigt gelassen, so daß bislang keine praktikablen Ergebnisse erzielt werden konnten. Die vorliegende Erfindung beinhaltet das erste Beispiel für chirale Diphosphonite im allgemeinen, mit dem sich Enantioselektivitäten von mehr als 99 % in der asymmetrischen Katalyse und damit für praktische Anwendungen interessante Selektivitäten erzielen lassen.

Der Grundgedanke der vorliegenden Erfindung beinhaltet chirale $C_2$-symmetrische Diphosphonite mit Ferrocen als Rückgrat (*backbone*), die im P/O-Heterocyclus entweder chirale $C_2$-symmetrische 1,2-Diole mit aliphatischem Grundgerüst oder axial chirale aromatische oder heteroaromatische Diole enthalten sowie deren Synthese. Die Erfindung schließt Metallkomplexe dieser Liganden und ihre Verwendung in der asymmetrischen Synthese mit ein. Liganden dieses Typs zeigen in der Hydrierung verschiedener prochiraler Olefine exzellente Enantioselektivitäten, sind jedoch deutlich einfacher und daher kostengünstiger darzustellen als bisher in der Literatur bekannte Systeme, die eine vergleichbar hohe Selektivität aufweisen (z.B. DuPHOS oder PennPHOS; M. J. Burk *et al., J. Am. Chem. Soc.* **1995,** *117*, 9375 bzw. X. Zhang *et al., Angew. Chem.* **1998,** *110*, 1203).

Im einzelnen umfaßt die Erfindung 1,1'-Ferrocenylendiphosphonite des Typs **I, II, III** und **IV.**

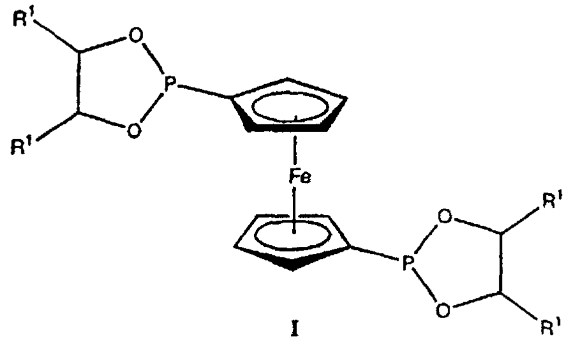

I

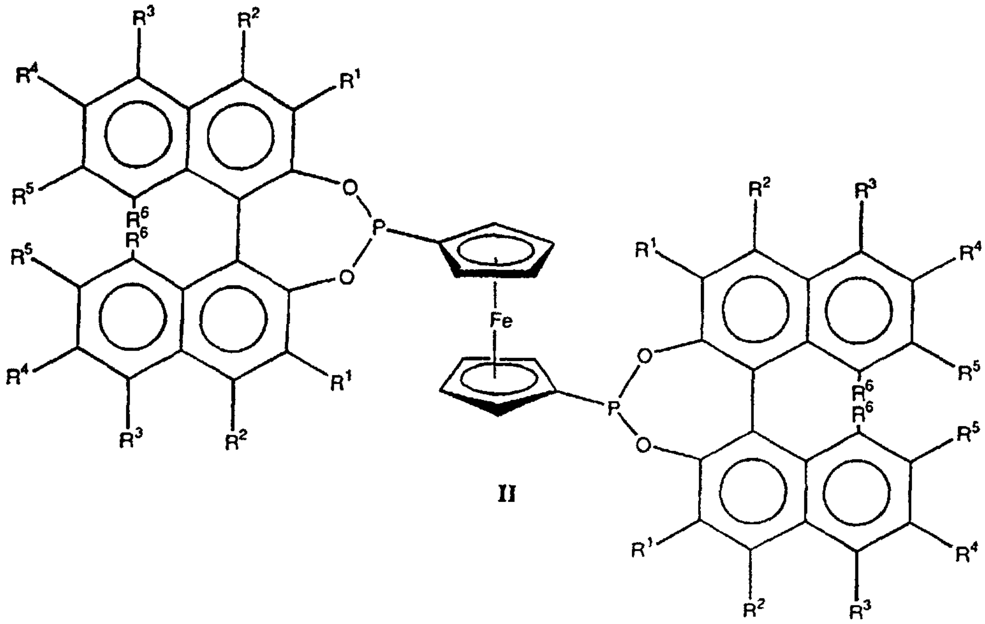

II

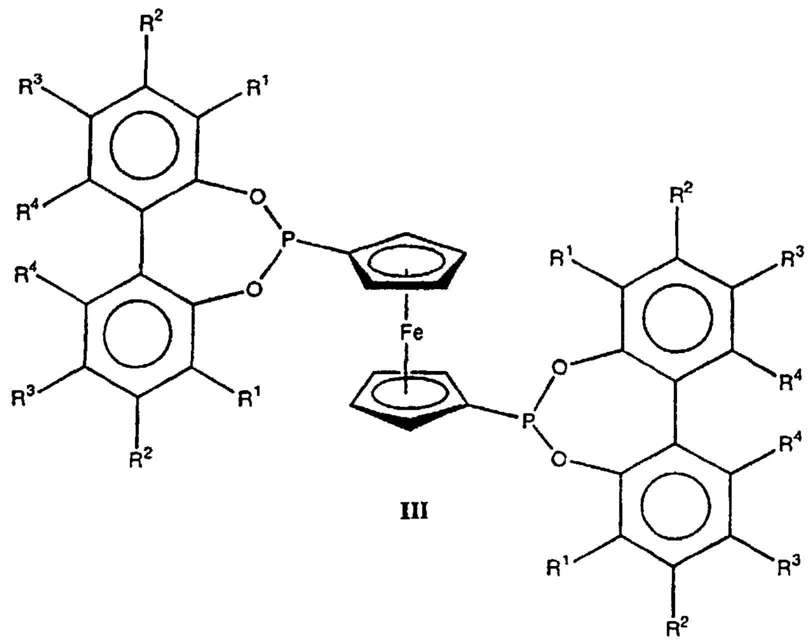

III

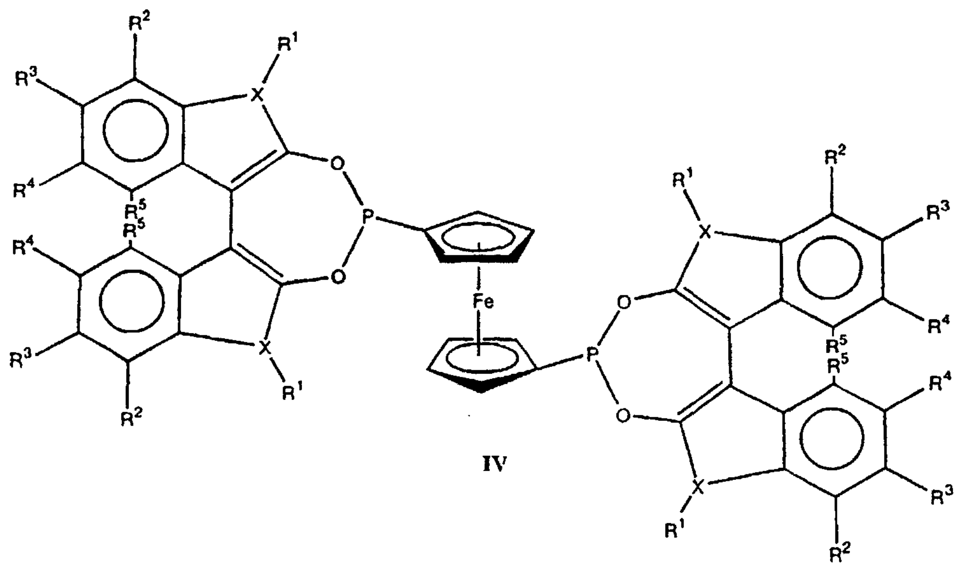

IV

Im Falle der Verbindungsklasse **I** sind die Bausteine $C_2$-symmetrische chirale Diole des Typs **V.**

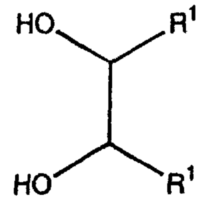

V

Der Rest $R^1$ kann dabei ein gesättigter Kohlenwasserstoff sein, der gegebenenfalls funktionalisiert sein kann, wie z. B. im Falle von 1,2-Diol-Einheiten von geschützten Kohlenhydraten oder geschützten Aminoalkoholen. Als Reste kommen auch nicht-aromatische, ungesättigte Kohlenwasserstoffe, ggf. funktionalisiert, sowie aromatische oder heteroaromatische Gruppen wie z.B. Phenyl, Naphthyl oder Pyridyl in Frage, die ihrerseits beliebig funktionalisiert sein können. Schließlich ist es möglich, daß die Reste aus Ester- oder Amidgruppen bestehen, wie z. B. $-CO_2CH_3$, $-CO_2C_2H_5$, $-CO_2$*i*-$C_3H_7$ bzw. $-CO[N(CH_3)_2]$, $-CO[N(C_2H_5)_2]$ oder -CO[N(*i*-$C_3H_7)_2]$, wobei die entsprechenden Diole **V** Weinsäurederivate darstellen.

Im Falle der Ligandenklasse **II** besteht der Sauerstoff-haltige Baustein aus Binaphthol **VI,** mit Resten $R^1$, $R^2$, $R^3$, $R^4$, $R^5$ und $R^6$, die unabhängig voneinander nachfolgende Gruppen darstellen können: Wasserstoff (H), gesättigte Kohlenwasserstoffe, gegebenenfalls auch funktionalisiert und/oder verbrückend (z. B. $R^1 + R^2 = -(CH_2)_4-$), aromatische oder heteroaromatische Gruppen, die auch funktionalisiert und/oder anelliert sein können und somit cyclische Reste darstellen (beispielsweise $R^1 + R^2$ = *ortho*-Phenylen; entspricht 4,4'-Dihydroxy-5,5'-bis-(phenanthryl)), nichtaromatische ungesättigte Kohlenwasserstoffe wie z.B. Alkinylgruppen -C≡CR, die auch funktionalisiert sein können, Silylgruppen wie z.B. $-SiMe_3$, Halogene (-Cl, -Br, -F, -I), Nitro- ($-NO_2$) oder Nitrilgruppen (-CN) außerdem Ester ($-CO_2R$), Amide (-C(O)NRR'), Amine (-NRR'), Ether (-OR), Sulfide (-SR) und Selenide (-SeR) in denen R und R' Wasserstoff, gesättigte oder nichtaromatische ungesättigte Kohlenwasserstoffe, gegebenenfalls auch funktionalisiert, oder aromatische Reste, gegebenenfalls auch funktionalisiert, sind. Insbesondere sind in der vorgestellten Erfindung alle Kombinationen der genannten Reste für $R^1$, $R^2$, $R^3$, $R^4$, $R^5$ und $R^6$ einschließlich aller $C_1$- und $C_2$-symmetrischen Substitutionsmuster des Binaphtholgrundkörpers enthalten. Ferner können einzelne oder mehrere Kohlenstoffatome des Binaphtholgerüstes durch Heteroatome wie z.B. Stickstoff ersetzt sein. Vorzugsweise dient Binaphthol ($R^1 = R^2 = R^3 = R^4 = R^5 = R^6 = H$) selbst als Baustein.

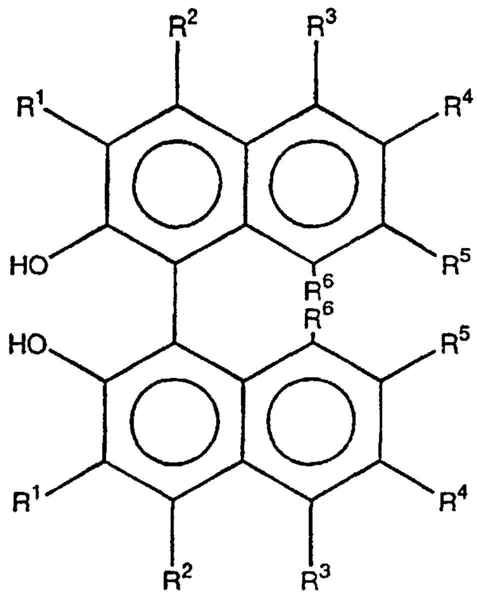

VI

Im Falle der Verbindungsklasse **III** ist der Dihydroxy-Baustein ein funktionalisiertes konfigurativ stabiles Biphenol **VII.** Konfigurative Stabilität hinsichtlich der axialen Chiralität ist dann gewährleistet, wenn $R^4 \neq H$ (E. L. Eliel, S. H. Wilen, L. N. Mander, *Stereochemistry of Organic Compounds,* Wiley, New York, **1994**). $R^1$ bis $R^4$ weisen hierbei dieselbe Variationsbreite wie die Reste $R^1$ bis $R^6$ im Falle der Verbindungsklasse **VI** auf. Vorzugsweise ist jedoch $R^1 = R^2 = H$ und $R^3 + R^4 = -(CH_2)_4-$ (2,2'-Dihydroxy-5,5'-6,6'-7,7'-8,8'-octahydro-1,1'-binaphthyl, D. J. Cram, *et al., J. Org. Chem.* **1978,** *43,* 1930).

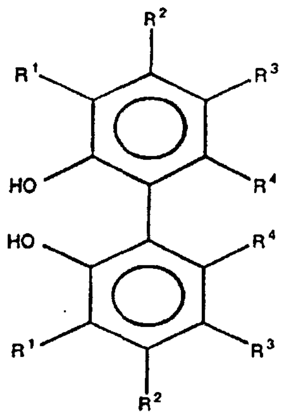

VII

Im Falle der Verbindungsklasse **IV** ist der Dihydroxy-Baustein ein funktionalisiertes, konfigurativ stabiles heteroaromatisches System **VIII,** welches sich vom 2,2'-Dihydroxy-3,3'-bis(indolyl) (X = N), 2,2'-Dihydroxy-3,3'-bis(benzo[b]thiophenyl) (X = S) oder 2,2'-Dihydroxy-3,3'-bis(benzo[b]furanyl) (X = O) ableitet. Auch in diesen Fällen weisen die Substituenten dieselbe Variationsbreite auf wie in **VI.** Der Substituent $R^1$ entfällt für die Fälle X = O und X = S.

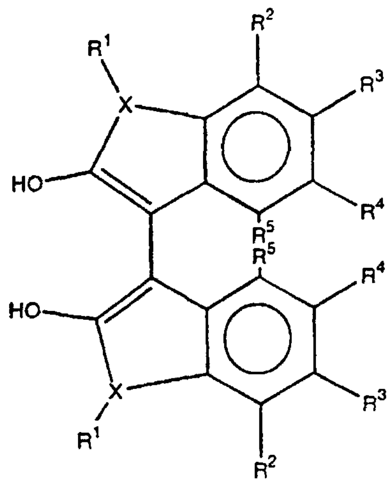

VIII

Die vorliegende Erfindung beinhaltet alle stereoisomeren Formen der Diole **V, VI, VII** und **VIII** als Bausteine.

Schema 1 zeigt den erfindungsgemäßen Syntheseweg der Liganden auf. In der ersten Stufe wird eine in der Literatur (J. J. Bishop, *et al., J. Organomet. Chem.* **1971,** *27*, 241) bekannte zweifache Lithiierung von Ferrocen mittels *n*-Butyllithium in Gegenwart von Tetramethylethylendiamin (TMEDA) vorgenommen, gefolgt von Phosphorylierung mit Phosphorchloriden wie z.B. $ClP[N(CH_3)_2]_2$ oder $ClP[N(C_2H_5)_2]_2$ unter Bildung der Verbindungsklasse **IX,** die im zweiten Schritt mit den Diolen **V, VI, VII** oder **VIII** unter Bildung der Liganden **I, II, III** bzw. **IV** umgesetzt werden.

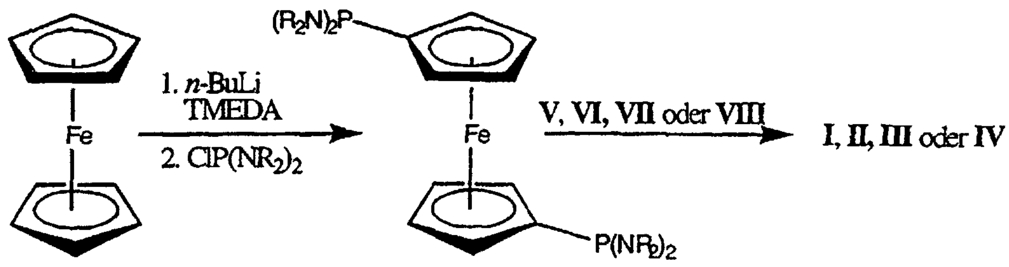


IX

Schema 1.

Eine Variante der Synthese schließt eine weitere Stufe ein, nämlich die Umsetzung von **IX** mit HCl unter Bildung von **X,** das dann mit den Diolen **V, VI, VII** oder **VIII** umgesetzt wird (Schema 2). In vielen Fällen erhöht sich dadurch die Gesamtausbeute.

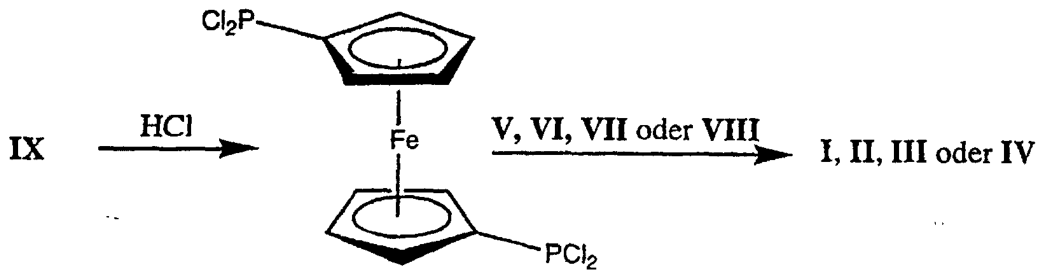


X

Schema 2.

Die Erfindung schließt auch die Bildung von neuen Metallkomplexen durch Reaktion der erfindungsgemäßen Liganden mit Übergangsmetallverbindungen ein, die üblicherweise im Falle von Diphosphinen eingesetzt werden, insbesondere Metalle der Gruppen VIIb, VIIIb und Ib des Periodensystems (s. z. B. B. Cornils, W. A. Herrmann, *Applied Homogeneous Catalysis with Organometallic Compounds*, Wiley-VCH, Weinheim, **1996;** R. Noyori, *Asymmetric Catalysis in Organic Synthesis*, Wiley, New York, 1994). Beispiele sind Rh-, Ru-, Ir-, Ni-, Pd - oder Cu-Komplexe des Typs **XI - XXXVIII** (hierbei steht cod für $\eta^2;\eta^2$-1,5-Cyclooctadien und cymol für $\eta^6$-1-*iso*-Propyl-4-methylbenzol).

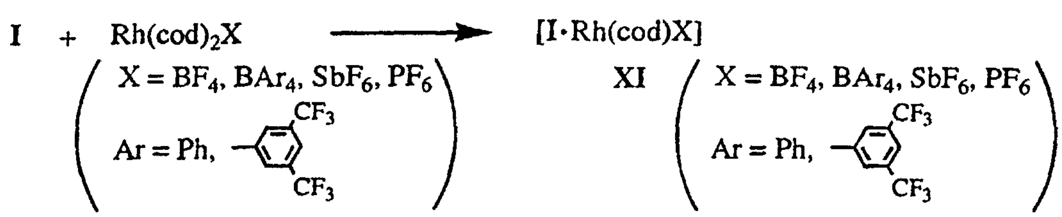

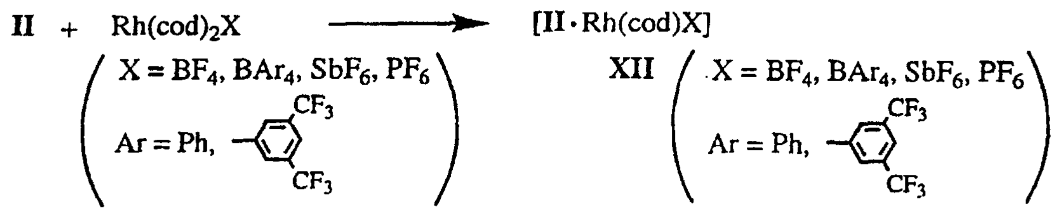


XII

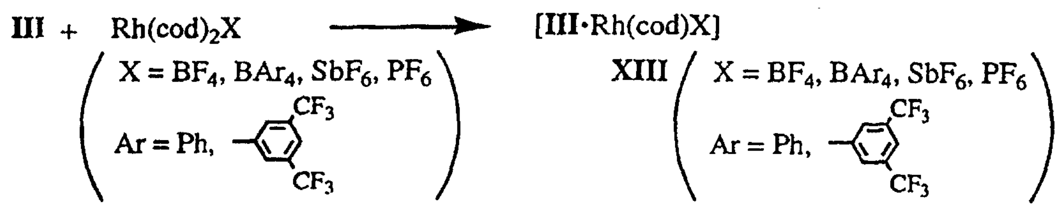


XIII

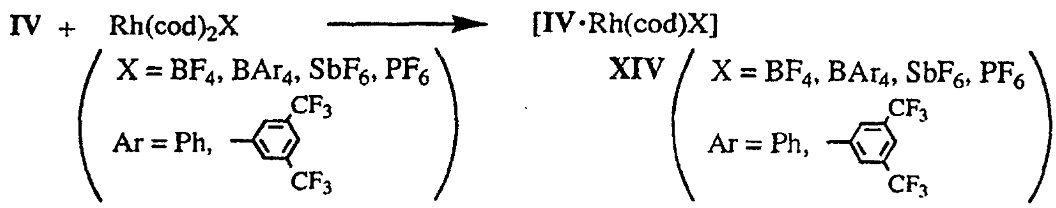


XIV

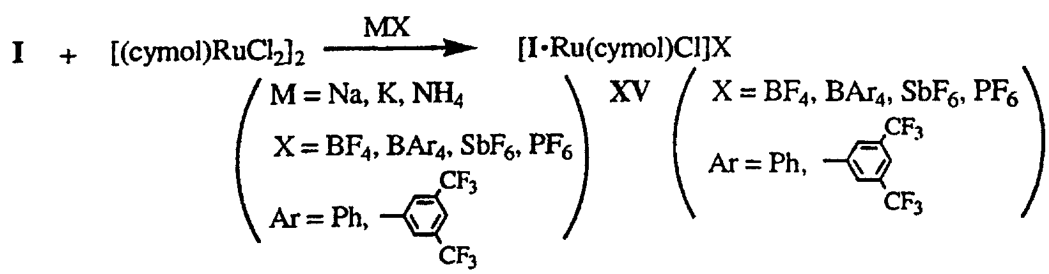


XV

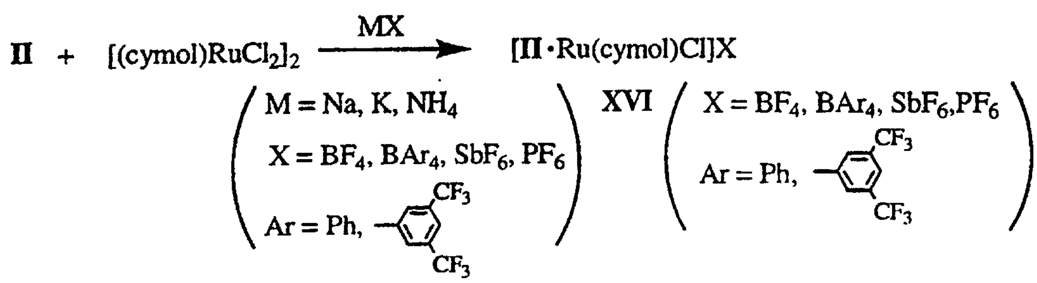


XVI

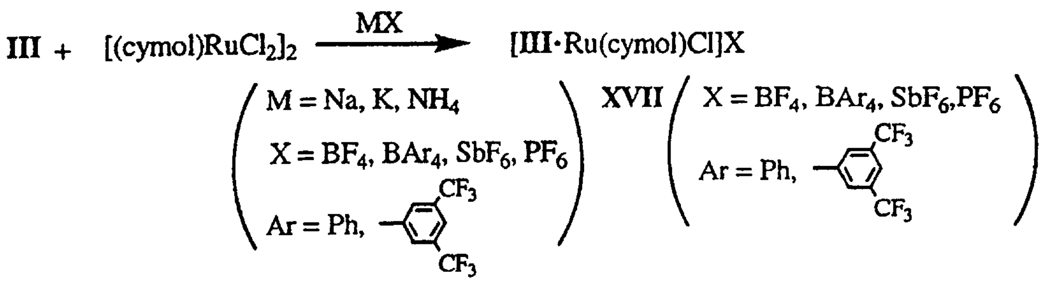
III + [(cymol)RuCl2]2
MX
[III·Ru(cymol)Cl]X
XVII
M = Na, K, NH4
X = BF4, BAr4, SbF6, PF6
Ar = Ph,
CF3
CF3
X = BF4, BAr4, SbF6,PF6
Ar = Ph,
CF3
CF3

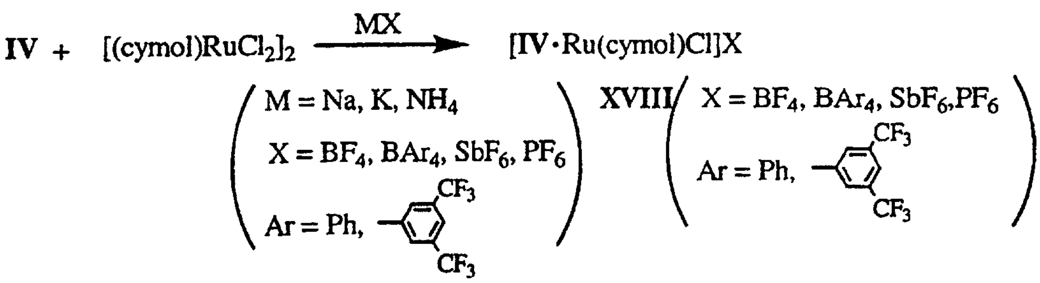
IV + [(cymol)RuCl2]2
MX
[IV·Ru(cymol)Cl]X
XVIII
M = Na, K, NH4
X = BF4, BAr4, SbF6, PF6
Ar = Ph,
CF3
CF3
X = BF4, BAr4, SbF6,PF6
Ar = Ph,
CF3
CF3

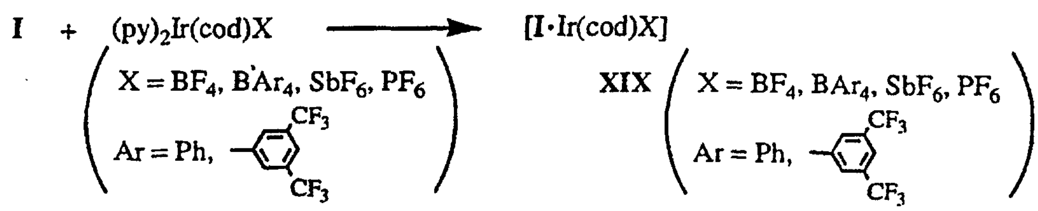
I + (py)2Ir(cod)X
[I·Ir(cod)X]
XIX
X = BF4, BAr4, SbF6, PF6
Ar = Ph,
CF3
CF3
X = BF4, BAr4, SbF6, PF6
Ar = Ph,
CF3
CF3

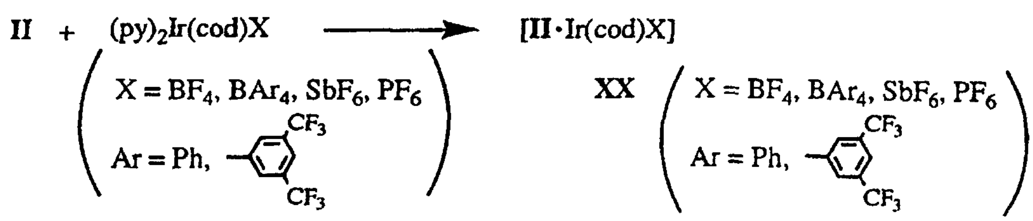
II + (py)2Ir(cod)X
[II·Ir(cod)X]
XX
X = BF4, BAr4, SbF6, PF6
Ar = Ph,
CF3
CF3
X = BF4, BAr4, SbF6, PF6
Ar = Ph,
CF3
CF3

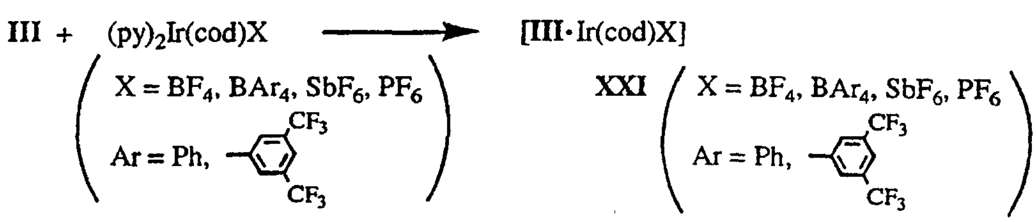
III + (py)2Ir(cod)X
[III·Ir(cod)X]
XXI
X = BF4, BAr4, SbF6, PF6
Ar = Ph,
CF3
CF3
X = BF4, BAr4, SbF6, PF6
Ar = Ph,
CF3
CF3

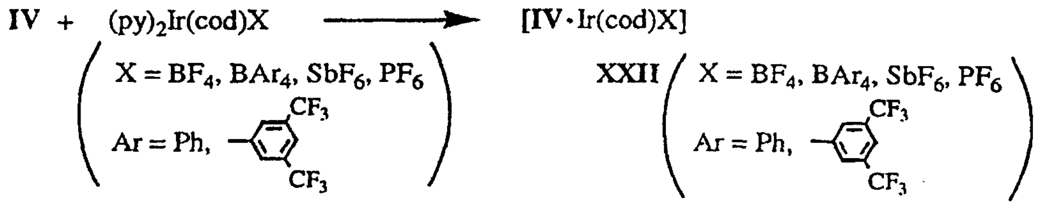

XXII

$I + Ni(cod)_2 \rightarrow [I \cdot Ni(cod)]$ **XXIII**

$II + Ni(cod)_2 \rightarrow [II \cdot Ni(cod)]$ **XXIV**

$III + Ni(cod)_2 \rightarrow [III \cdot Ni(cod)]$ **XXV**

$IV + Ni(cod)_2 \rightarrow [IV \cdot Ni(cod)]$ **XXVI**

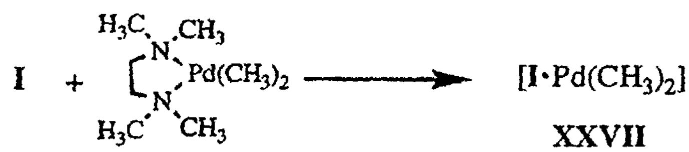

XXVII

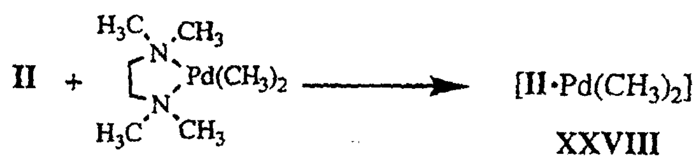

XXVIII

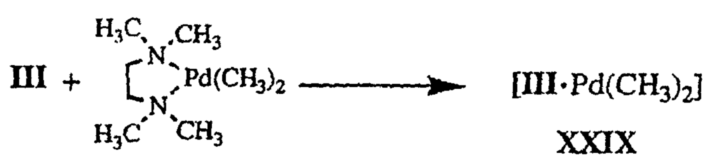

XXIX

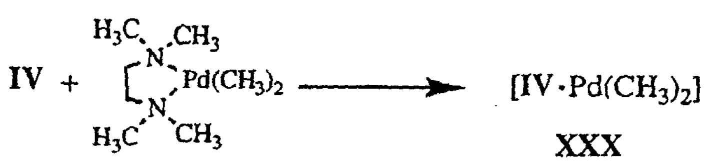

XXX

**I** + CuOTf $\longrightarrow$ [**I**·CuOTf]
(Tf = $SO_2CF_3$)    **XXXI** (Tf = $SO_2CF_3$)

**II** + CuOTf $\longrightarrow$ [**II**·CuOTf]
(Tf = $SO_2CF_3$)    **XXXII** (Tf = $SO_2CF_3$)

**III** + CuOTf $\longrightarrow$ [**III**·CuOTf]
(Tf = $SO_2CF_3$)    **XXXIII** (Tf = $SO_2CF_3$)

**IV** + CuOTf $\longrightarrow$ [**IV**·CuOTf]
(Tf = $SO_2CF_3$)    **XXXIV** (Tf = $SO_2CF_3$)

**I** + $Cu(OTf)_2$ $\longrightarrow$ [**I**·$Cu(OTf)_2$]
(Tf = $SO_2CF_3$)    **XXXV** (Tf = $SO_2CF_3$)

**II** + $Cu(OTf)_2$ $\longrightarrow$ [**II**·$Cu(OTf)_2$]
(Tf = $SO_2CF_3$)    **XXXVI** (Tf = $SO_2CF_3$)

**III** + $Cu(OTf)_2$ $\longrightarrow$ [**III**·$Cu(OTf)_2$]
(Tf = $SO_2CF_3$)    **XXXVII** (Tf = $SO_2CF_3$)

**IV** + $Cu(OTf)_2$ $\longrightarrow$ [**IV**·$Cu(OTf)_2$]
(Tf = $SO_2CF_3$)    **XXXVIII** (Tf = $SO_2CF_3$)

Schließlich beinhaltet die Erfindung auch die Anwendung der erfindungsgemäßen Metallkomplexe als Katalysatoren in der asymmetrischen Katalyse wie z. B. Hydrierung, Hydroformylierung, Hydrocyanierung, Hydrosilylierung, Hydrovinylierung, Hydroborierung und Kupfer-katalysierter 1,4-Addition. Beispiele hierfür sind die asymmetrischen Hydrierungen von Dimethylitaconat **XXXIX**, 2-Acetamidomethylacrylat **XL,** (*Z*)-2-Acetamidozimtsäure **XLI** und ihr Methylester **XLII,** α-Acetamidostyrol **XLIII** und *N*-(1-Phenylethyliden)anilin **XLIV,** die unter Verwendung der erfindungsgemäßen Metallkomplexe mit sehr hohen chemischen Ausbeuten und Enantioselektivitäten durchführbar sind. Ebenso die Hydroborierung von Sytyrol **VL** und die Kupfer-katalysierte 1,4-Addition and 2-Cyclohexen-1-on **VLI** oder 2-Cyclohepten-1-on **VLII.** Diese Ergebnisse sind von großer praktischer Relevanz und machen diese Verbindungen auch für

industrielle Anwendungen interessant.

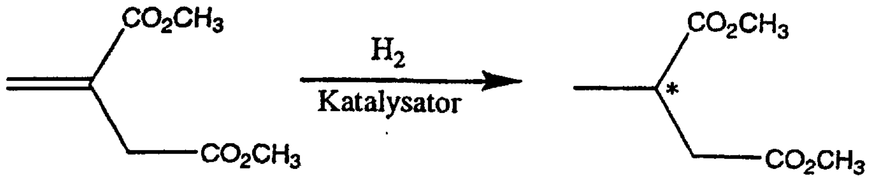


XXXIX

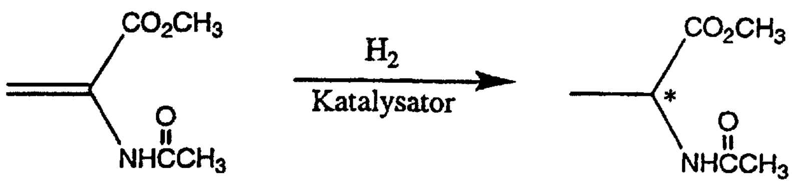


XL

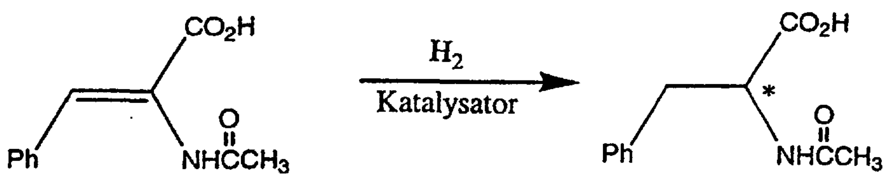


XLI

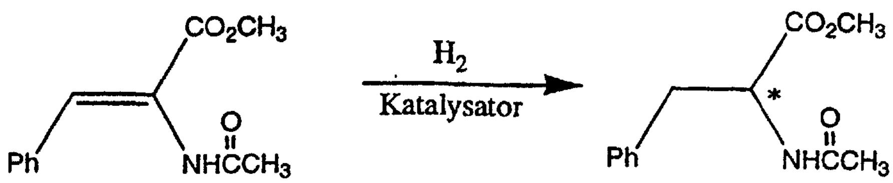


XLII

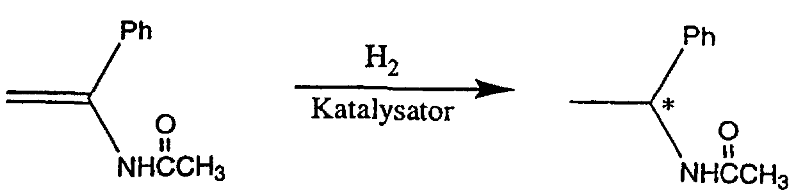


XLIII

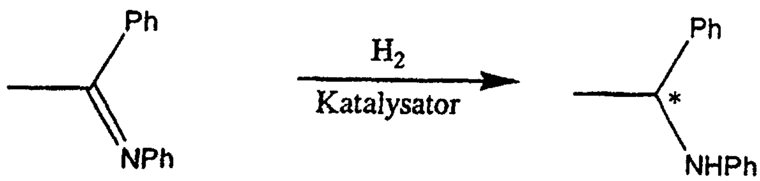


**XLIV**

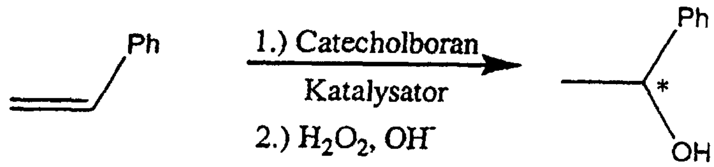


**VL**

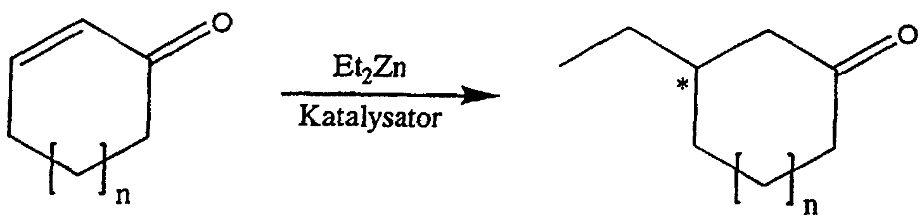


**n =1, VLI**
**n = 2, VLII**

**Beispiel 1.** Synthese von 1,1'-Bis[bis(diethylamino)phosphino]ferrocen (**IX**, R = Et)

30.0g (0.161 mol) Ferrocen werden bei Raumtemperatur in 600 ml abs. Hexan vorgelegt. Innerhalb von 40 min wird hierzu ein Gemisch aus 250 ml (0.40 mol) 1.60 M *n*-Butyllithium-Lösung in Hexan und 63.0 ml (48.5 g, 0.417 mol) abs. *N,N,N',N'*-Tetramethylethylendiamin bei Raumtemperatur zugetropft. Man läßt über Nacht bei Raumtemperatur rühren. Der ausgefallene orangefarbene Feststoff wird über eine P4-Umkehrfritte abfiltriert und gründlich mit Pentan gewaschen bis das Filtrat farblos bleibt. Der Filtrationsrückstand wird anschließend im Ölpumpenvakuum getrocknet. Man erhält 37.7 g (0.12 mol, 75 %) *N,N,N',N'*-Tetramethylethylendiamino-1,1'-dilithioferrocen als feinpulvriges, orangefarbenes Pulver.

11.7 g (37.2 mmol) des Pulvers werden bei -78 °C in 300 ml abs. THF suspendiert. Hierzu wird innerhalb von 1 h eine Lösung von 20.0 ml (92.3 mmol) Bis(diethylamino)chlorphosphin in 50 ml abs. THF zugetropft. Man läßt auf Raumtemperatur auftauen und rührt weitere 15 h. Die nunmehr rotbraun gefärbte Lösung wird vom Lösungsmittel befreit und der ölige Rückstand in 300 ml abs. Pentan aufgenommen. Durch Filtration über eine P4-Fritte mit Celite 545® als Filtrierhilfe wird das ungelöste Lithiumchlorid abgetrennt. Das klare Filtrat wird wiederum vollständig vom Lösungsmittel befreit. Der ölige rotbraune Rückstand wird einer fraktionierten Destillation bei $10^{-6}$ mbar unterworfen. Das Produkt geht unter diesen Bedingungen bei einer Kopftemperatur von 175-190 °C über. Man erhält 18.4 g (34.7 mmol, 93 % berechnet auf eingesetztes *N,N,N',N'*-Tetramethylethylendiamino-1,1'-dilithioferrocen) eines sehr viskosen, rotbraunen Öls. Analytik: $^1$H-NMR ($d_6$-$C_6H_6$, 300 MHz): 4.13 (t) $J$ = 1.8 Hz [4H], 4.10 (m) [4H], 2.81 (m) [16H], 0.81 (t) $^3J_{H\text{-}H}$= 7.2 Hz [24H]; $^{13}$C-{$^1$H}-NMR ($d_6$-$C_6H_6$, 75 MHz): 82.1 (d) $J_{C\text{-}P}$ = 11.9, 72.9 (dd) $J_{C\text{-}P}$ = 2.6 Hz, 9.8 Hz, 72.5 (dd) $J_{C\text{-}P}$ = 2.3 Hz, 4.4 Hz, 43.1 (d) $^2J_{C\text{-}P}$ = 17.4 Hz, 15.2 (d) $^3J_{C\text{-}P}$ = 3.2 Hz; $^{31}$P-NMR ($d_6$-$C_6H_6$, 121 MHz): 91.6 (s); MS (EI, pos. Ionen): *m/z* = 534 [$M^+$] (30 %), 463 [$M^+$-$C_4H_9N$] (14 %), 391 (72 %), 320 (100 %), 247 (25 %), 195 (25 %), 128 (11 %), 104 (10 %); HRMS (EI, pos. Ionen): gef.: 534.270322 ± 0.000625 erw.: 534.270361; IR (Kapillar): $\tilde{\nu}$ ($cm^{-1}$) =

3097 (w), 2965 (s), 2929 (m-s), 2850 (m-s), 1461 (m), 1373 (s), 1342 (w), 1291 (m-w), 1187 (s), 1100 (w), 1072 (m-w), 1023 (s), 1012 (s), 908 (s), 792 (m-s), 662 (s-m).

**Beispiel 2.** Synthese von 1,1'-Bis(dichlorphosphino)ferrocen (**X**)

8.63 g (16.3 mmol) 1,1'-Bis[bis(diethylamino)phosphino]ferrocen (**IX**, R = Et) werden bei -78 °C in 400 ml abs. Diethylether vorgelegt. Hierzu wird innerhalb von 2 h eine Lösung von 40 ml einer 3.9 M HCl in abs. Diethylether in 150 ml abs. Diethylether zugetropft. Nach beendeter Zugabe läßt man auf Raumtemperatur erwärmen und rührt über Nacht. Man erhält eine orange Lösung mit darin suspendiertem farblosen Feststoff, der durch Filtration über eine P4-Fritte mit Celite 545® als Filtrierhilfe abgetrennt wird. Das klare orange Filtrat wird anschließend vollständig vom Lösungsmittel befreit. Der orange Feststoff wird anschließend aus 30 ml abs. Toluol/Pentan-Gemisch (1:1) umkristallisiert. Man erhält 5.94 g (15.3 mmol) des Produktes **X** (94 %) in Form grober, nadelförmiger Kristalle. Analytik: $^1$H-NMR ($d_6$-$C_6H_6$, 300 MHz): 4.66 ppm (s); $^{13}$C-{$^1$H}-NMR ($d_6$-$C_6H_6$, 75 MHz): 82.3 (d) $J_{C-P}$ = 55.4 Hz, 74.8 (t) $J_{C-P}$ = 2.9 Hz, 72.8 (m); $^{31}$P-NMR ($d_6$-$C_6H_6$, 121 MHz): 163.7 (s); MS (EI, pos. Ionen): *m/z* = 386 [$M^+$] (49 %), 351 [$M^+$-Cl] (17 %), 258 (5 %), 223 (5 %), 159 (24 %), 130 (100 %), 95 (58 %), 69 (19 %); EA: C: 30.69 % (ber. 30.97 %), P: 16.13 % (ber. 15.97 %), H: 2.15 % (ber. 2.08 %).

**Beispiel 3.** Direkte Synthese von 1,1'-Bis[dichlorphosphino]ferrocen (**X**) ohne Isolierung von Zwischenstufen.

40.69g (0.2186 mol) Ferrocen werden bei Raumtemperatur in 400 ml abs. Hexan vorgelegt. Innerhalb von 6 h wird hierzu ein Gemisch aus 342 ml (0.547 mol) 1.60 M *n*-Butyllithium-Lösung in Hexan und 82.0 ml (63.6 g, 0.547 mol) abs. *N,N,N',N'*-Tetramethylethylendiamin bei Raumtemperatur zugetropft. Man läßt 18 h bei Raumtemperatur rühren. Die überstehende Lösung wird über eine Immersionsfritte abfiltriert und der orange Filtrationsrückstand in 400 ml abs. THF suspendiert. Bei -78 °C wird innerhalb von 4 h eine Lösung von 95.0 ml (94.6 g, 0.481 mol) Bis(diethylamino)chlorphosphin in 200 ml abs. THF zugetropft. Man läßt auf Raumtemperatur auftauen und rührt weitere 15 h. Die nunmehr rotbraun gefärbte Lösung wird auf -78 °C gekühlt und 390 ml einer 5.6 M HCl in Diethylether werden innerhalb von 5 h zugetropft. Nach Erwärmen auf Raumtemperatur wird das Lösungsmittel vollständig abkondensiert und der Rückstand in 2 L Diethylether aufgenommen und über eine P4-Fritte mit Celite 545® als Filtrierhilfe wird vom Ungelösten abfiltriert. Das klare Filtrat wird wiederum vollständig vom Lösungsmittel befreit. Der rotbraune Feststoff wird zuletzt aus 200 ml Toluol / Pentan 1:1 bei -20 °C umkristallisiert. Man erhält nach dem Trocknen im Ölpumpenvakuum 52.82 g eines grobkristallinen Feststoffs (0.136 mol, 62 %) Analytik: entspricht (siehe Beispiel 2).

**Beispiel 4.** Synthese von (*R,R*)-1,1'-Bis{dinaphtho[1,2-d, 1,2 f][1,3,2]dioxaphosphepin-8-yl}ferrocen (**II**, $R^1$ = $R^2$ = $R^3$ = $R^4$ = $R^5$ = $R^6$ = H)

1.28 g (3.30 mmol) 1,1'-Bis(dichlorphosphino)ferrocen (**X**) werden zusammen mit 1.89 g (6.60 mmol) (*R*) -Binaphthol (> 99.9 % *ee*) in 250 ml abs. Toluol gelöst und 36 h lang unter Rückfluß erhitzt. Anschließend wird das Lösungsmittel vollständig entfernt und der Rückstand in 150 ml siedendem Toluol gelöst. Nach dem Abkühlen auf Raumtemperatur wird vom Ungelösten durch Filtration über eine P4-Fritte mit Celite 545® als Filtrierhilfe abgetrennt. Das klare, orange Filtrat wird auf 25 ml eingeengt und mit 80 ml abs. Pentan überschichtet. Über Nacht fällt ein feinkristalliner orangebrauner Feststoff aus, welcher abfiltriert und im Ölpumpenvakuum getrocknet wird. Man erhält 2.70 g (2.98 mmol, 90 %) eines feinkristallinen orangebraunen Feststoffes. Das Produkt enthält 1 mol Toluol pro Mol Substanz. Analytik: $^1$H-NMR ($d_2$-$CH_2Cl_2$, 300 MHz): 7.88-7.72 (m) [6H], 7.64 (s) [1H], 7.61 (s) [1H], 7.44 (s) [1H], 7.41 (s) [1H], 7.37-7.23 (m) [8H], 7.22-7.12 (m) [6H], 7.11-7.02 (m) [3H], 6.81 (s) [1H], 6.78 (s) [1H], 4.63 (m) [2H], 4.50 (s) [2H], 4.23 (m) [2H], 3.64 (m) [2H], 3.64 (s) [3H] PhC**H**$_3$ (Auswertung mit Toluol); $^{13}$C-{$^1$H}-NMR ($d_2$-$CH_2Cl_2$, 75 MHz): 149.3 8 (s), 148.3 (s), 132.0 (s), 131.7 (s), 130.7 (s), 130.2 (s), 128.5 (s), 127.6 (s), 127.5 (s), 127.3 (s), 125.80 (s), 125.78 (s), 125.2 (s), 124.4 (s), 124.1 (s), 123.9 (s), 123.7 (m), 122.6 (s), 121.4 (s), 120.8 (s), 76.1 (m), 72.9 (m), 72.3 (t) *J* = 4.1 Hz, 71.5 (s), 69.7 (s) (Auswertung ohne Toluol); $^{31}$P-NMR ($d_2$-$CH_2Cl_2$, 121 MHz): 190.8 (s); MS (EI, pos. Ionen): *m/z* = 814 [$M^+$] (100 %), 499 (7 %), 435 (14 %), 419 (12 %), 268 (10 %), 167 (12 %); IR (KBr): $\tilde{\nu}$ ($cm^{-1}$) = 3056 (w), 1617 (w-m), 1586 (m), 1505 (m), 1462 (m-s), 1430 (w), 1228 (s) νAr-O, 948 (s) νP-O, 820 (s), 798 (m-s), 780 (m-s), 751 (s), 684 (m), 636 (m-w), 573 (m), 552 (m), 497 (m); EA: C: 75.39 % (75.50 % ber.), H: 4.58 % (4.45 % ber.).

Das erhaltene Rohprodukt kann auch aus Dichlormethan umkristallisiert werden und fällt dabei in Form eines feinkristallinen orangen Feststoffs an (enthält 1.5 mol Dichlormethan pro Mol Substanz; Analytik entspricht).

**Beispiel 5.** Synthese von (*S,S*)-1,1'-Bis{dinaphtho[1,2-d, 1,2-f][1,3,2]dioxaphosphepin-8-yl}ferrocen (**II**, $R^1$ = $R^2$ = $R^3$ = $R^4$ = $R^5$ = $R^6$ = H)

Unter Verwendung von (*S*)-Binaphthol wird die sonst gleiche Vorschrift wie in Beispiel 3 angewandt. Man erhält 2.70 g (2.98 mmol, 90 %; berechnet als Toluol-Addukt) des (*S,S*)-Produkts. Analytik: NMR, MS und IR wie beim Beispiel 3.

**Beispiel 6.** Synthese von (*R,R*)-1,1'-Bis(dinaphtho[1,2-d, 1,2-f][1,3,2]dioxaphosphepin-8-yl)-ferrocen (**II**, R1 = R2 = R3 = R4 = R5 = R6 = H) augehend von 1,1'-Bis[bis(diethylamino)phosphino]ferrocen (**IX**, R = Et)

1.000 g stark saures Aluminiumoxid werden im Ölpumpenvakuum ausgeglüht. Nach dem Erkalten werden 1.471 g ( 2.773 mmol) 1,1'-Bis[bis(diethylamino)phosphino]ferrocen (**IX**, R = Et) und 1.710 g (5.546 mmol) (*R*)-Binaphthol und 75 ml abs. Toluol zugegeben. Das Gemisch wird 20 Tage zum Rückfluß erhitzt. Nach dem Abkühlen wird vom Ungelösten abfiltriert und das Filtrat vom Lösungsmittel befreit. Man erhält 1.011 g (1.115 mmol, 40 %) eines orangen

Feststoffs dessen Analytik nach Umkristallisation entspricht (siehe Beispiel 3).

**Beispiel 7.** Synthese von (*R,R*)-1,1'-Bis{3,3'-dimethyl-dinaphtho[1,2-d, 1,2-f]-[1,3,2]dioxaphosphepin-8-yl} ferrocen (**II**, R1 = $CH_3$, R2 = R3 = R4 = R5 = R6 = H)

0.772 g (1.99 mmol) 1,1'-Bis(dichlorphosphino)ferrocen (**X**) werden zusammen mit 1.407 g (3.981 mmol) (*R*)-3,3'-Dimethyl-binaphthol-Hemibenzolat (> 99.9 % *ee*) in 50 ml abs. Toluol gelöst und 23 Tage lang unter Rückfluß erhitzt. Anschließend wird das Lösungsmittel vollständig entfernt und der Rückstand in 10 ml Toluol gelöst. Nach dem Abkühlen auf Raumtemperatur wird vom Ungelösten abfiltriert und das klare, orange mit 40 ml abs. Pentan überschichtet. Man erhält das gewünschte Produkt in Form eines teilweise kristallinen orangebrauner Feststoffs, welcher abfiltriert und im Ölpumpenvakuum getrocknet wird. Man erhält 1.542 g (1.601 mmol, 80 %) eines orangebraunen Feststoffes. Das Produkt enthält 1 mol Toluol pro Mol Substanz. $^1$H-NMR ($d_2$-$CH_2Cl_2$, 300 MHz): 7.78-7.63 (m) [6H], 7.47 (s) [2H], 7.34-7.20 (m) [4H], 7.18-6.98 (m) [13H], 4.76 (m) [2H], 4.55 (m) [2H], 4.25 (m) [2H], 3.57 (m) [2H], 2.46 (s) [3H], 2.24 (s) [3H] PhC**H**$_3$, 1.79 (s) [3H] (Auswertung mit Toluol); $^{13}$C-{$^1$H]-NMR ($d_2$-$CH_2Cl_2$, 75 MHz): 148.2 (s), 147.7 (s), 137.2 (s) ($PhCH_3$), 130.9 (s), 130.58 (s), 130.55 (s), 130.2 (s), 129.8 (s), 129.3 (s), 129.2 (s), 128.3 (s), 128.2 (s), 127.4 (s), 126.8 (s), 126.7 (s), 125.8 (s), 125.7 (s), 124.5 (s), 124.4 (s), 124.2 (s), 124.1 (s),123.9 (s), 123.8 (s), 122.6 (s), 21.5 (s) ($PhCH_3$), 17.7 (s), 17.4 (s); ($d_1$-$CHCl_3$, 75 MHz): ca. 77.6 (m, X-Kern eines ABX-Spinsystems), 73.4 (m, X-Kern eines ABX-Spinsystems), 72.6 (m), 72.4 (s), 70.2 (s) (Auswertung mit Toluol); $^{31}$P-NMR ($d_2$-$CH_2Cl_2$, 121 MHz): 187.2 (s). MS (ESI, pos. Ionen): *m/z* = 870 [$M^+$] (100 %), 527 (9 %), 463 (33%), 447 (20 %), 435 (11 %), 296 (13 %), 280 (10 %), 167 (21 %).

Durch Verwendung von *o*-Xylol als Lösungsmittel kann die Reaktionszeit auf 4 Tage herabgesetzt werden (1.60 mmol Ansatzgröße, 62 % Ausbeute nach Umkristallisation, Analytik entspricht).

**Beispiel 8.** Synthese von (*R,R,R,R*)-1,1'-Bis(4,5-diphenyl-1,3,2-dioxapholan-2-yl)ferrocen (**I,** $R^1$ = $R^2$ = Ph)

651 mg (3.04 mmol) (*R,R*)-Hydrobenzoin werden zusammen mit 1.0 ml abs. Triethylamin (6.63 mmol) bei -78 °C in 150 ml abs. THF vorgelegt. Innerhalb von 2 h wird hierzu eine Lösung von 425 mg (0.80 mmol) 1,1'-Bis(dichlorphosphino)ferrocen (**X**) in 25 ml abs. THF zugetropft. Anschließend lässt man unter Rühren über Nacht im Kühlbad langsam auftauen. Die erhaltene Lösung wird vollständig vom Lösungsmittel befreit und der orange Feststoff in 200 ml abs. Diethylether aufgenommen. Durch Filtration über eine P4-Fritte mit Celite 545® als Filtrierhilfe wird vom Ungelösten abgetrennt. Das klare orangefarbene Filtrat wird vollständig vom Lösungsmittel befreit. Man erhält einen orangefarbenen Feststoff. Analytik: $^1$H-NMR ($d_8$-THF, 300 MHz): 7.40 (s) [8H], 7.36-730 (m) [6H], 7.29-7.23 (m) [4H], 7.22-7.11 (m) [6H], 5.05 (d) $J_{H-P}$ = 8.1 Hz [2H], 4.95 (d) $J_{H-P}$ = 9 Hz [2H], 4.82-4.60 (m) [8H]; $^{31}$P-NMR ($d_8$-THF, 121 MHz): 181.7 (s).

**Beispiel 9.** Synthese von (*R,R*)-1,1'-Bis(dinaphtho[1,2-d, 1,2-f][1,3,2]dioxaphosphepin-8-yl}ferrocen-$\eta^2$;$\eta^2$-1,5-cyclooctadien-rhodium(I)-tetrafluoroborat (**XII**, $R^1$ = $R^2$ = $R^3$ = $R^4$ = $R^5$ = $R^6$ = H)

14.7 ml (0.24 mmol) einer 16.4 mM Lösung von $Rh(cod)_2BF_4$ in abs. Dichlormethan werden bei -78 °C vorgelegt. Mit Hilfe einer Spritzenpumpe wird eine Lösung von 219 mg (0.242 mmol) (*R,R*)-1,1'-Bis{dinaphtho[1,2-d, 1,2-f] [1,3,2]-dioxaphosphepin-8-yl}ferrocen-Toluol-Addukt (**II**, $R^1$ = $R^2$ = $R^3$ = $R^4$ = $R^5$ = $R^6$ = H) in 20 ml abs. Dichlormethan tropfenweise hinzugegeben. Nach beendeter Zugabe läßt man auf Raumtemperatur erwärmen und rührt über Nacht. Die erhaltene Lösung wird auf 7 ml eingeengt und unter Rühren wird schnell 50 ml abs. Pentan zugesetzt. Man erhält eine hellgelbe Lösung mit einem darin suspendierten orangefarbenen Feststoff. Die überstehende Lösung wird abfiltriert und der erhaltene Feststoff im Ölpumpenvakuum getrocknet. Man erhält 245 mg (0.20 mmol, berechnet als $CH_2Cl_2$-Addukt) eines orangefarbenen, pulvrigen Feststoffs (berechnet als $CH_2Cl_2$-Addukt, Ausbeute: 85 %). Analytik: $^1$H-NMR (d-$CHCl_3$, 300 MHz): 8.19 (s) [4H], 7.99 (s) [1H], 7.96 (s) [1H], 7.83 (s) [1H], 7.80 (s) [1H], 7.73 (s) [1H], 7.70 (s) [1H], 7.53-7.35 (m) [4H], 7.35-7.21 (m) [8H], 7.05 (s) [1H], 7.02 (s) [1H], 6.41 (t) $J_{H-P}$ = 7 Hz [2H], 5.10 (m) [2H], 4.59 (m) [2H], 4.19 (d) $J_{H-P}$ = 1 Hz, 3.94 (m) [2H], 3.84 (s) [2H], 2.83-2.20 (m) [6H], 1.85-1.58 (m) [2H]; $^{13}$C-{$^1$H} -NMR ($d_2$-$CH_2Cl_2$, 75 MHz): 133.3 (s), 133.1 (s), 132.8 (s), 132.5 (s), 132.4 (s), 131.5 (s), 129.6 (s), 129.5 (s), 128.1 (s), 127.7 (s) [2C], 127.3 (s), 127.0 (s), 126.6 (s), 124.3 (s), 123.4 (s), 122.0 (s), 121.8 (s), 112.2 (q) $J_{C-P}$ = 5 Hz, 106.6 (q) $J_{C-P}$ = 6.5 Hz, 78.1 (t) $J_{C-P}$ = 6.5 Hz, 77.8 (t) $J_{C-P}$ = 16.2 Hz, 74.7 (s), 74.4 (s), 34.1 (s), 27.8 (s); $^{31}$P-NMR ($d_2$-$CH_2Cl_2$, 81 MHz): 169.4 (d) $^1J_{P-Rh}$ = 212.7 Hz; MS (ESI, pos. Ionen): *m/z* = 1025 [1112-$BF_4$], 917 [$M^+$-COD]; IR (KBr): $\tilde{\nu}$ (cm$^{-1}$) = 3056 (w), 2947 (w), 2920 (w), 2920 (w), 2879 (w), 2829 (w), 1586 (m), 1507 (m), 1462 (m), 1424 (m-w), 1361 (w), 1321 (m), 1222 (s), 1185 (s-m), 1068 (s), 1051 (s), 1033 (s), 944 (s), 825 (s), 806 (s), 771 (m), 691 (m), 558 (m-s); EA: C: 5910 % (ber. 59.80 %), P: 5.41 % (ber. 5.17 %), H: 3.95 % (ber. 3.87 %), Rh: 8.98 % (ber. 8.59 %), Cl: 5.49 % (ber. 5.92 %) berechnete Werte bezogen auf das Mono-$CH_2Cl_2$-Addukt.

**Beispiel 10.** Synthese von (*S,S*)-1,1'-Bis{dinaphtho[1,2-d, 1,2f][1,3,2]dioxaphosphepin-8-yl}ferrocen-$\eta^2$;$\eta^2$-1,5-cyclooctadien-rhodium(I)-tetrafluoroborat (**XII**, $R^1$ = $R^2$ = $R^3$ = $R^4$ = $R^5$ = $R^6$ = H)

Unter Verwendung von (*S,S*)-1,1'-Bis{dinaphtho[1,2-d, 1,2-f][1,3,2]dioxaphosphepin-8-yl}ferrocen wird die sonst gleiche Vorschrift wie im Beispiel 6 an gewandt. Man erhält 246 mg (0.20 mmol) eines orangefarbenen Feststoffs (85 %).

**Beispiel 11.** Synthese von (*R,R*)-1,1'-Bis{dinaphtho[1,2-d, 1,2-f][1,3,2]dioxaphosphepin-8-yl}ferrocen-$\eta^2$;$\eta^2$-1,5-cyclooctadien-iridium(**I**)-hexafluorophosphat (**XX**, $R^1$ = $R^2$ = $R^3$ = $R^4$ = $R^5$ = $R^6$ = H)

324 mg (0.36 mmol) (*R,R*)-1,1'-Bis{dinaphtho[1,2-d, 1,2 f][1,3,2]dioxaphosphe pin -8-yl}ferrocen-Toluol-Addukt **(II,** $R^1 = R^2 = R^3 = R^4 = R^5 = R^6 = H$) werden in 20 ml abs. Dichlormethan gelöst und mit Hilfe einer Spritzenpumpe tropfenweise zu einer auf -78 °C gekühlten Lösung von 212 mg (0.35 mmol) Bis(pyridin)- $\eta^2$;$\eta^2$-1,5-cyclooctadien-iridium(I)-hexafluorophosphat in 20 ml abs. Dichlormethan gegeben. Man lässt langsam über Nacht auf Raumtemperatur erwärmen und entfernt das Lösungsmittel vollständig. Der erhaltene grünliche Feststoff wird mit 20 ml abs. Diethylether gewaschen. Man erhält 400 mg eines grünlichen Feststoffs (0.32 mmol, 91 %). Analytik: $^1$H-NMR ($d_2$-$CH_2Cl_2$, 300 MHz): 8.14 (s) [1H], 8.11 (s) [1H], 8.03-7.93 (m) [4H], 7.88 (s) [1H], 7.85 (s) [1H], 7.82 (s) [1H], 7.77 (s) [1H], 7.52-7.40 (m) [6H], 7.35-7.17 (m) [ca. 6H], 7.01 (s) [1H], 6.98 (s) [1H], 6.28 (m) [2H], 5.03 (m) [2H], 4.64 (m) [2H], 4.24 (m) [2H], 3.89 (m) [2H], 3.59 (m) [2H], 2.47-2.22 (m) [5H], 1.80-1.49 (m) [3H]; $^{31}$P-NMR ($d_2$-$CH_2Cl_2$, 121 MHz): 136.5 (s).

**Beispiel 12.** Synthese von (*R,R*)-1,1'-Bis{dinaphtho[1,2-d, 1,2-f][1,3,2]dioxaphosphepin-8-yl}ferrocen-chloro-$\eta^6$-1,4-cymol-ruthenium(II)-chlorid (**XVI**, $R^1 = R^2 = R^3 = R^4 = R^5 = R^6 = H$)

162 mg (0.18 mmol) (*R,R*)-1,1'-Bis{dinaphtho[1,2-d, 1,2-f][1,3,2]dioxaphosphepin-8-yl}ferrocen-Toluol-Addukt (**II,** $R^1 = R^2 = R^3 = R^4 = R^5 = R^6 = H$) werden in 20 ml abs. Dichlormethan gelöst und mit Hilfe einer Spritzenpumpe tropfenweise zu 6.0 ml (0.09 mmol) einer auf -78 °C gekühlten 15.5 mM Lösung Bis[$\eta^6$-1,4-Cymol-ruthenium(II)-dichlorid] (0.19 mmol Ru) in abs. Dichlormethan gegeben. Man läßt langsam über Nacht auf Raumtemperatur erwärmen und entfernt das Lösungsmittel vollständig. Man erhält nach dem Trocknen im Ölpumpenvakuum einen rotbraunen Feststoff.

**Beispiel 13.** Synthese von (*R,R*)-1,1'-Bis{dinaphtho[1,2-d, 1,2-f][1,3,2]dioxaphosphepin-8-yl}ferrocenkupfer (I)-triflat (**XXXII**, $R^1 = R^2 = R^3 = R^4 = R^5 = R^6 = H$)

53.1 mg 90%iges CuOTf · 0.5 PhH (Kupfer(I)-triflat-Hemibenzolat, Tf = -$SO_2CF_3$) (0.19 mmol) werden über Nacht bei Raumtemperatur in abs. Dichlorethan gerührt. Bei 10 °C werden 13 ml (0.20 mmol) einer 15 mM Lösung von (*R,R*)-1,1'-Bis{dinaphtho[1,2-d, 1,2-f][1,3,2]dioxaphosphepin-8-yl ferrocen-Toluol-Addukt **(II,** $R^1 = R^2 = R^3 = R^4 = R^5 = R^6 = H$) in abs. Dichlorethan über eine Spritzenpumpe langsam zu der CuOTf-Lösung getropft. Die Lösung wird über Nacht bei Raumtemperatur gerührt und anschließend über eine P4-Fritte filtriert. Das klare dunkel-orange Filtrat wird bei Raumtemperatur mit 30 ml abs. Pentan überschichtet. Nach 3 Tagen bei Raumtemperatur wird die überstehende Lösung abfiltriert und der zurückbleibende Feststoff im Ölpumpenvakuum getrocknet. Man erhält einen grünlich-beigen Feststoffs.

**Beispiel 14.** Vorschrift für die enantioselektive Hydrierung von Dimethylitaconat mit *in situ* hergestelltem Katalysator (*R,R*)-1,1'-Bis{dinaphtho[1,2-d, 1,2-f][1,3,2]-dioxaphosphepin-8-yl}ferrocen-$\eta^2$;$\eta^2$-1,5-cyclooctadien-rhodium(**I**) tetrafluoroborat (**XII,** $R^1 = R^2 = R^3 = R^4 = R^5 = R^6 = H$)

8.0 ml einer 0.125 M Substratlösung (Dimethylitaconat) in Dichlormethan werden in einem Rundkolben mit Seithahn vorgelegt. Hierzu wird 1.0 ml einer 1.0 mM Lösung von $Rh(cod)_2BF_4$ in Dichlormethan und anschließend 1.1 ml einer 1.0 mM Lösung von (*R,R*)-1,1'-Bis{dinaphtho[1,2-d, 1,2-f][1,3,2]dioxaphosphepin-8-yl}ferrocen (**II**, $R^1 = R^2 = R^3 = R^4 = R^5 = R^6 = H$) in Dichlormethan zugegeben. Die Lösung wird nun mit Wasserstoff gesättigt indem drei mal bis zum leichten Sieden des Lösungsmittels evakuiert und mit Wasserstoff begast wird. Zuletzt wird die Lösung unter 1.3 bar Wasserstoffdruck gesetzt und für 20 h bei Raumtemperatur gerührt. Zur gaschromatographischen Analyse des Produktgemisches werden 2 ml der so erhaltenen Lösung über 125 mg Silica (70 - 230 mesh, Aktivitätsstufe I) filtriert. Bei quantitativer Hydrierung wird ein Enantiomerenüberschuss (*ee*) von 99.6 % zugunsten des *R*-konfigurierten Produkts 2-Methylbernsteinsäuredimethylester gemessen.

**Beispiel 15.** Vorschrift für die enantioselektive Hydrierung von Dimethylitaconat mit präformierten Metallkomplexen als Katalysator (*R,R*)-1,1'-Bis{dinaphtho[1,2-d, 1,2-f][1,3,2]-dioxaphosphepin-8-yl} ferrocen-$\eta^2$;$\eta^2$-1,5-cyclooctadienrhodium(I)-tetrafluoroborat (**XII**, $R^1 = R^2 = R^3 = R^4 = R^5 = R^6 = H$)

8.0 ml einer 0.125 M Substratlösung (Dimethylitaconat) in Dichlormethan werden in einem Rundkolben mit Seithahn vorgelegt. Hierzu wird 1.0 ml einer 1.0 mM Lösung von (*R,R*)-1,1'-Bis{dinaphtho[1,2-d, 1,2-f][1,3,2]dioxaphosphepin-8-yl}ferrocen-$\eta^2$;$\eta^2$-1,5-cyclooctadien-rhodium(I)-tetrafluoroborat (**XII**, $R^1 = R^2 = R^3 = R^4 = R^5 = R^6 = H$) in Dichlormethan und anschließend 1.0 ml Dichlormethan zugegeben. Die Lösung wird nun mit Wasserstoff gesättigt, indem drei mal bis zum leichten Sieden des Lösungsmittels evakuiert und mit Wasserstoff begast wird. Zuletzt wird die Lösung unter 1.3 bar Wasserstoffdruck gesetzt und für 20 h bei Raumtemperatur gerührt. Zur gaschromatographischen Analyse des Produktgemisches werden 2 ml der so erhaltenen Lösung über 125 mg Silica (70 - 230 mesh, Aktivitätsstufe I) filtriert. Bei quantitativer Hydrierung wird ein Enantiomerenüberschuss (*ee*) von 99.6 % zugunsten des (*R*)-konfigurierten Produkts 2-Methylbernsteinsäuredimethyleser gemessen.

**Beispiel 16.** Vorschrift für die enantioselektive Hydrierung von Dimethylitaconat mit *in situ* hergestelltem Katalysator (*S,S*)-1,1'-Bis{dinaphtho[1,2-d, 1,2-f][1,3,2]-dioxaphosphepin-8-yl}ferrocen-$\eta^2$;$\eta^2$-1,5-cyclooctadien-rhodium (I) tetrafluoroborat (**XII**, $R^1 = R^2 = R^3 = R^4 = R^5 = R^6 = H$)

8.0 ml einer 0.125 M Substratlösung (Dimethylitaconat) in Dichlormethan werden in einem Rundkolben mit Seithahn vorgelegt. Hierzu wird 1.0 ml einer 1.0 mM Lösung von $Rh(cod)_2BF_4$ in Dichlormethan und anschließend 1.1 ml einer 1.0 mM Lösung von (*S,S*)-1,1'-Bis{dinaphtho[1,2-d, 1,2-f][1,3,2]dioxaphosphepin-8-yl}ferrocen (**II**, $R^1 = R^2 = R^3 = R^4 = R^5 = R^6 = H$) in Dichlormethan zugegeben. Die Lösung wird nun mit Wasserstoff gesättigt indem drei mal

bis zum leichten Sieden des Lösungsmittels evakuiert und mit Wasserstoff begast wird.

Zuletzt wird die Lösung unter 1.3 bar Wasserstoffdruck gesetzt und für 20 h bei Raumtemperatur gerührt. Zur gaschromatographischen Analyse des Produktgemisches werden 2 ml der so erhaltenen Lösung über 125 mg Silica (70 - 230 mesh, Aktivitätsstufe I) filtriert. Bei quantitativer Hydrierung wird ein Enantiomerenüberschuss *(ee)* von 99.6 % zugunsten des (*S*)-konfigurierten Produkts 2-Methylbernsteinsäuredimethylester gemessen.

**Beispiel 17.** Vorschrift für die enantioselektive Hydrierung von 2-Acetamidomethylacrylat mit *in situ* hergestelltem Katalysator (*R,R*)-1,1'-Bis{dinaphtho-[1,2-d, 1,2-f][1,3,2]-dioxaphosphepin-8-yl}ferrocen-$\eta^2$;$\eta^2$-1,5-cyclooctadien-rhodium(I) tetrafluoroborat (**XII**, $R^1 = R^2 = R^3 = R^4 = R^5 = R^6 = H$)

8.0 ml einer 0.125 M Substratlösung (2-Acetamidomethylacrylat) in Dichlormethan werden in einem Rundkolben mit Seithahn vorgelegt. Hierzu wird 1.0 ml einer 1.0 mM Lösung von Rh(cod)2BF4 in Dichlormethan und anschließend 1.1 ml einer 1 mM Lösung von (*R,R*)-1,1'-Bis{dinaphtho[1,2-d, 1,2-f][1,3,2]dioxaphosphepin-8-yl}ferrocen (**II**, $R^1 = R^2 = R^3 = R^4 = R^5 = R^6 = H$) in Dichlormethan zugegeben. Die Lösung wird nun mit Wasserstoff gesättigt, indem drei mal bis zum leichten Sieden des Lösungsmittels evakuiert und mit Wasserstoff begast wird. Zuletzt wird die Lösung unter 1.3 bar Wasserstoffdruck gesetzt und für 20 h bei Raumtemperatur gerührt. Zur gaschromatographischen Analyse des Produktgemisches werden 2 ml der so erhaltenen Lösung über 125 mg Silica Silica (70 - 230 mesh, Aktivitätsstufe I) filtriert. Bei quantitativer Hydrierung wird ein Enantiomerenüberschuss (*ee*) von 99.5 % zugunsten des (*R*)-konfigurierten Produkts *N*-Acylalaninmethylester gemessen.

**Beispiel 18.** Wie Beispiel 14 , jedoch mit 2-Acetamidoarylsäuremethylester als Edukt und (*R*)-konfiguriertem *N*-Acetylalaninmethylester als Produkt (quantitativer Umsatz; *ee* = 99.5%).

**Beispiel 19.** Vorschrift für die enantioselektive Hydrierung von (*Z*)-2-Acetamidozimtsäuremethylester mit *in situ* hergestelltem Katalysator (*R,R*)-1,1'-Bis{dinaphtho[1,2-d, 1,2-f][1,3,2]-dioxaphosphepin-8-yl}ferrocen-$\eta^2$;$\eta^2$-1,5-cyclooctadien-rhodium(I) tetrafluoroborat (**XII**, $R^1 = R^2 = R^3 = R^4 = R^5 = R^6 = H$).

In einem Rundkolben mit Seithahn werden 1.0 ml einer 1.0 mM Lösung von $Rh(cod)_2BF_4$ in Dichlormethan und anschließend 1.1 ml einer 1 mM Lösung von (*R,R*)-1,1'-Bis{dinaphtho[1,2-d, 1,2-f][1,3,2]dioxaphosphepin-8-yl} ferrocen (**II**, $R^1 = R^2 = R^3 = R^4 = R^5 = R^6 = H$) in Dichlormethan vorgelegt. Hierzu werden 8.0 ml einer 0.125 M Substratlösung (von (Z)-2-Acetamidozimtsäuremethylester) in Dichlormethan zugegeben. Die Lösung wird nun mit Wasserstoff gesättigt, indem drei mal bis zum leichten Sieden des Lösungsmittels evakuiert und mit Wasserstoff begast wird. Zuletzt wird die Lösung unter 1.3 bar Wasserstoffdruck gesetzt und für 20 h bei Raumtemperatur gerührt. Zur gaschromatographischen Analyse des Produktgemisches werden 2 ml der so erhaltenen Lösung über 125 mg Silica Silica (70 - 230 mesh, Aktivitätsstufe I) filtriert. Bei quantitativer Hydrierung zu *N*-Acylphenylalaninmethylester wird ein Enantiomerenüberschuss (*ee*) von 99 % gemessen. Die Bestimmung des Enantiomerenüberschuss (*ee*) erfolgte über HPLC an chiraler stationärer Phase.

**Beispiel 20.** Vorschrift für die enantioselektive Hydrierung von (*Z*)-2-Acetamidozimtsäure mit *in situ* hergestelltem Katalysator (*R,R*)-1,1'-Bis{dinaphtho[1,2-d, 1,2-f][1,3,2]-dioxaphosphepin-8-yl}ferrocen-$\eta^2$;$\eta^2$-1,5-cyclooctadien-rhodium(I) tetrafluoroborat (**II**, $R^1 = R^2 = R^3 = R^4 = R^5 = R^6 = H$).

In einem 30 ml Schlenk-Gefäß werden 1.0 ml einer 1.0 mM Lösung von $Rh(cod)_2BF_4$ in Dichlormethan vorgelegt und anschließend 1.1 ml einer 1 mM Lösung von (*R,R*)-1,1'-Bis{dinaphtho[1,2-d, 1,2-f][1,3,2]dioxaphosphepin-8-yl }ferrocen (**II**, $R^1 = R^2 = R^3 = R^4 = R^5 = R^6 = H$) in Dichlormethan zugegeben. Hierzu werden 8.0 ml einer 0.125 M Substratlösung ((Z)-2-Acetamidozimtsäure) in Dichlormethan/*iso*-Propanol 3:1 gegeben. Die Lösung wird in einen 50 ml V4A-Stahl-Autoklaven mit Tefloneinsatz und Manometeraufsatz überführt und unter 20 bar Wasserstoffdruck gesetzt und für 20 h bei Raumtemperatur gerührt. Nach dem Entspannen des Autoklaven wird die gesamte Lösung vollständig vom Lösungsmittel befreit und zur Bestimmung des Umsatzes $^1$H-NMR spektroskopisch analysiert. Die Bestimmung des Enantiomerenüberschusses erfolgt über HPLC an chiraler stationärer Phase. Bei quantitativer Hydrierung zu *N*-Acylphenylalanin wird ein Enantiomerenüberschuss (*ee*) von 99 % gemessen.

**Beispiel 21.** Vorschrift für die enantioselektive Hydrierung von *N*-(1-Phenylethyliden)anilin mit *in situ* hergestelltem Katalysator (*R,R*)-1,1'-Bis{dinaphtho[1,2-d, 1,2-f][1,3,2]-dioxaphosphepin-8-yl}ferrocen-$\eta^2$;$\eta^2$-1,5-cyclooctadien-iridium(I)hexafluorophosphat (**XX**, $R^1 = R^2 = R^3 = R^4 = R^5 = R^6 = H$, $X = PF_6$).

In einem 30 ml Schlenk-Gefäß werden 1 ml einer 1.0 mM Lösung von $(py)_2Ir(cod)PF_6$ in Dichlormethan vorgelegt und anschließend 1.1 ml einer 1 mM Lösung von (*R,R*)-1,1'-Bis{dinaphtho[1,2-d, 1,2-f][1,3,2]dioxaphosphepin-8-yl}ferrocen (**II**, $R^1 = R^2 = R^3 = R^4 = R^5 = R^6 = H$) in Dichlormethan zugegeben. Hierzu werden 8.0 ml einer 0.125 M Substratlösung ((*Z*)-2-Acetamidozimtsäure) in Dichlormethan gegeben. Die Lösung wird in einen 50 ml V4A-Stahl-Autoklaven mit Tefloneinsatz und Manometeraufsatz überführt und unter 100 bar Wasserstoffdruck gesetzt und für 24 h bei Raumtemperatur gerührt. Nach dem Entspannen des Autoklaven werden 2 ml der so erhaltenen Lösung über 125 mg Silica Silica (70 - 230 mesh, Aktivitätsstufe I) filtriert. Die Bestimmung des Umsatzes erfolgt gaschromatographisch und der Enantiomerenüberschuss wird über HPLC an chiraler stationärer Phase ermittelt. Bei quantitativer Hydrierung zu *N*-Phenyl-1-phenylethylamin wird ein Enantiomerenüberschuss *(ee)* von 63 % gemessen.

**Beispiel 22.** Vorschrift für die enantioselektive Hydrierung von $\alpha$-Acetamidostyrol mit *in situ* hergestelltem Katalysator (*R,R*)-1,1'-Bis{dinaphtho[1,2-d, 1,2-f]-[1,3,2]dioxaphosphepin-8-yl}ferrocen-$\eta^2$;$\eta^2$-1,5-cyclooctadien-rho-

dium(I) tetrafluoroborat (**XII**, $R^1 = R^2 = R^3 = R^4 = R^5 = R^6 = H$).

In einem 30 ml Schlenk-Gefäß werden 2.0 ml einer 1.0 mM Lösung von $Rh(cod)_2BF_4$ in Dichlormethan vorgelegt und anschließend 2.2 ml einer 1 mM Lösung von (*R,R*)-1,1'-Bis{dinaphtho[1,2-d, 1,2-f][1,3,2]dioxaphosphepin-8-yl}ferrocen (**II**, $R^1 = R^2 = R^3 = R^4 = R^5 = R^6 = H$) in Dichlormethan zugegeben. Hierzu werden 8.0 ml einer 0.125 M Substratlösung ($\alpha$-Acetamidostyrol) in Dichlormethan gegeben. Die Lösung wird in einen 50 ml V4A-Stahl-Autoklaven mit Tefloneinsatz und Manometeraufsatz überführt und unter 80 bar Wasserstoffdruck gesetzt und für 20 h bei Raumtemperatur gerührt. Nach dem Entspannen des Autoklaven wird die gesamte Lösung vollständig vom Lösungsmittel befreit und zur Bestimmung des Umsatzes $^1$H-NMR spektroskopisch analysiert. Die Bestimmung des Enantiomerenüberschusses erfolgt über HPLC an chiraler stationärer Phase. Bei quantitativer Hydrierung zu *N*-Acyl-1-phenylethylamin wird ein Enantiomerenüberschuss *(ee)* von 96 % gemessen.

**Beispiel 23.** Wie Beispiel 22, jedoch unter Verwendung von nur 1.0 ml einer 1.0 mM Lösung von $Rh(cod)_2BF_4$ in Dichlormethan und 1.1 ml einer 1 mM Lösung von (*R,R*)-1,1'-Bis{dinaphtho[1,2-d, 1,2-f][1,3,2]dioxaphosphepin-8-yl} ferrocen (**II**, $R^1 = R^2 = R^3 = R^4 = R^5 = R^6 = H$) in Dichlormethan führte bei >99 % Umsatz zu einem Enantiomerenüberschuss *(ee)* von > 92 %.

**Beispiel 24.** Vorschrift für die enantioselektive Hydroborierung von Styrol mit *in situ* hergestelltem Katalysator (*R,R*)-1,1'-Bis{dinaphtho[1,2-d, 1,2-f][1,3,2]-dioxaphosphepin-8-yl}ferrocen-$\eta^2$;$\eta^2$-1,5-cyclooctadien-rhodium(I) tetrafluoroborat (**XII,** $R^1 = R^2 = R^3 = R^4 = R^5 = R^6 = H$).

In einem 30 ml Schlenk-Gefäß werden 1.0 ml einer 10 mM Lösung von $Rh(cod)_2BF_4$ in Dichlormethan vorgelegt und anschließend 1.1 ml einer 10 mM Lösung von (*R,R*)-1,1'-Bis{dinaphtho[1,2-d, 1,2-f][1,3,2]dioxaphosphepin-8-yl}ferrocen (**II**, $R^1 = R^2 = R^3 = R^4 = R^5 = R^6 = H$) in Dichlormethan zugegeben. Nach dem Entfernen des Lösungsmittels werden 2.0 ml einer 0.5 M Lösung von Styrol in Dimethoxyethan (DME) zugegeben und die erhaltene Lösung auf -80 °C gekühlt. Zu dieser Lösung wird 1.0 ml einer 3 M Lösung von Catecholboran in DME gegeben und das Reaktionsgemisch bei -80 °C gerührt.

Durch Zugabe von 1 ml Methanol und erwärmen auf Raumtemperatur wird die Reaktion nach 22 h gestoppt. Der Ansatz wird auf 0 °C gekühlt und nacheinander mit 2 ml 3 M wäßriger NaOH und 0.5 ml 30 % wäßrigem $H_2O_2$ versetzt. Nach dem Erwärmen auf Raumtemperatur werden 25 ml dest. Wasser und 30 ml Dichlormethan zugesetzt und die Phasen getrennt. Die wäßrige Phase wird drei Mal mit je 25 ml Dichlormethan extrahiert. Die organischen Phasen werden vereinigt und je zwei Mal mit je 30 ml 1 M wäßriger NaOH und danach gesättigter wäßriger $NH_4Cl$ ausgeschüttelt. Zuletzt wird über Magnesiumsulfat getrocknet und vom Lösungsmittel befreit. Die Bestimmung von Umsatz und Enantiomerenüberschuss (*ee*) erfolgt gaschromatographisch. Bei quantitativem Umsatz konnte ein Enantiomerenüberschuss (*ee*) von 87 % und eine Selektivität (gegenüber der Bildung von 2-Phenylethanol) von 97 % zugunsten des (*R*)-1-Phenylethanol bestimmt werden.

**Beispiel 25.** Wie Beispiel 24 jedoch bei einer Temperatur von - 30 °C führte bei 100 % Umsatz und 83 % Ausbeute (1-Phenylethanol, Bestimmt gegen n-Tetradecan als internem GC-Standard unter Berücksichtigung von FID-Response-Faktoren) zu einem Enantiomerenüberschuss (*ee*) von 84 % und einer Selektivität (gegenüber der Bildung von 2-Phenylethanol) von 93 % zugunsten des (*R*)-1-Phenylethanol.

**Beispiel 26.** Wie Beispiel 24 jedoch bei Raumtemperatur führte bei 100 % Umsatz und 79 % Ausbeute (1-Phenylethanol, Bestimmt gegen *n*-Tetradecan als internem GC-Standard unter Berücksichtigung von FID-Response-Faktoren) zu einem Enantiomerenüberschuss (*ee*) von 72 % und einer Selektivität (gegenüber der Bildung von 2-Phenylethanol) von 90 % zugunsten des (*R*)-1-Phenylethanol.

**Beispiel 27.** Wie Beispiel 24 jedoch unter Verwendung des präformierten Metallkomplex (*R,R*)-1,1'-Bis{dinaphtho[1,2-d, 1,2-f][1,3,2]-dioxaphosphepin-8-yl}ferrocen-$\eta^2$;$\eta^2$-1,5-cyclooctadien-rhodium(I)-tetrafluoroborat (**XII**, $R^1 = R^2 = R^3 = R^4 = R^5 = R^6 = H$) als Katalysator (vorgelegt als 10 mM Lösung (1.0 ml) in Dichlormethan anstelle der *in situ* erzeugten Lösung) führte bei vollständigem Umsatz und 85 % Ausbeute (1-Phenylethanol, Bestimmt gegen *n*-Tetradecan als internem GC-Standard unter Berücksichtigung von FID-Response-Faktoren) zu einem Enantiomerenüberschuss (*ee*) von 88 % und einer Selektivität (gegenüber der Bildung von 2-Phenylethanol) von 96 % zugunsten des (*R*)-1-Phenylethanol.

**Beispiel 28.** Vorschrift für die enantioselektive 1,4-Addition von Diethylzink an 2-Cyclohexen-1-on mit *in situ* hergestelltem Katalysator Bis{(*R,R*)-1,1'bis{dinaphtho[1,2-d, 1,2-f][1,3,2]-dioxaphosphepin-8-yl}ferrocen}-kupfer(II)-triflat (**XXXVI,** $R^1 = R^2 = R^3 = R^4 = R^5 = R^6 = H$).

In einem 30 ml Schlenk-Gefäß werden 1.0 ml einer 10 mM Lösung von $Cu(OTf)_2$ in Tetrahydrofuran bei Raumtemperatur vorgelegt und anschließend 2 ml einer 10 mM Lösung von (*R,R*)-1,1'-Bis{dinaphtho[1,2-d, 1,2-f][1,3,2] dioxaphosphepin-8-yl}ferrocen (**II**, $R^1 = R^2 = R^3 = R^4 = R^5 = R^6 = H$) in Tetrahydrofuran zugegeben. Nach 30 min Rühren bei Raumtemperatur wird die erhaltene Lösung auf -30 °C gekühlt und 1.0 ml einer 1 M Lösung von 2-Cyclohexen-1-on in THF zugegeben. Zu dieser Lösung wird 1.0 ml einer 1.5 M Lösung von Diethylzink in Tetrahydrofuran gegeben und das Reaktionsgemisch bei -30 °C gerührt.

Nach 20 h wird die Reaktion durch Zugabe von 10 ml ca. 1.3 M wäßriger HCl gestoppt und der Ansatz auf 0 °C gebracht. Nach Zugabe von 20 ml dest. Wasser und 30 ml Dichlormethan werden die Phasen bei Raumtemparatur getrennt und

die wäßrige Phase drei Mal mit je 20 ml Dichlormethan ausgeschüttelt. Nach dem Trocknen über Magnesiumsulfat wird das Lösungsmittel vollständig entfernt und das Rohprodukt in 2 ml Dichlormethan aufgenommen. Die Bestimmung von Umsatz und Enantiomerenüberschuss (*ee*) erfolgte gaschromatographisch und ergab bei vollständigem Umsatz einen *ee* von 95 %. Das Produkt aus einer 1,2-Addition wurde nicht gebildet.

**Beispiel 29.** Wie Beispiel 28 jedoch mit 2-Cyclohepten-1-on (95 % Reinheit) anstelle des 2-Cyclohexen-1-on führte bei 92 % Umsatz zu einem Enantiomerenüberschuss (*ee*) von 84 %. Das Produkt aus einer 1,2-Addition wurde nicht gebildet.

## Patentansprüche

1. Chirale $C_2$-symmetrische Diphosphonite mit Ferrocen als Rückgrat (backbone), **dadurch gekennzeichnet, daß** sie im P/O-Heterocyclus entweder chirale $C_2$-symmetrische 1,2-Diole mit aliphatischem Grundgerüst oder axial chirale aromatische oder heteroaromatische Diole enthalten.

2. Diphosphonite nach Anspruch 1, die enantio- und diastereomerenrein sind.

3. Diphosphonite nach Anspruch 1 und 2, wobei deren Struktur der allgemeinen Formel I entspricht, wobei $R^1$ einen gesättigten Kohlenwasserstoff, gegebenenfalls funktionalisiert, einen nichtaromatischen ungesättigten Kohlenwasserstoff, gegebenenfalls funktionalisiert, eine aromatische oder heteroaromatische Gruppe gegebenenfalls funktionalisiert, einen Ester ($-CO_2R$) oder ein Amid (-C(O)NRR') darstellt, in denen R und R' Substituenten repräsentieren, die Wasserstoff, gesättigte oder nichtaromatische ungesättigte Kohlenwasserstoffe, gegebenenfalls funktionalisiert, oder aromatische Reste, gegebenenfalls funktionalisiert, sind:

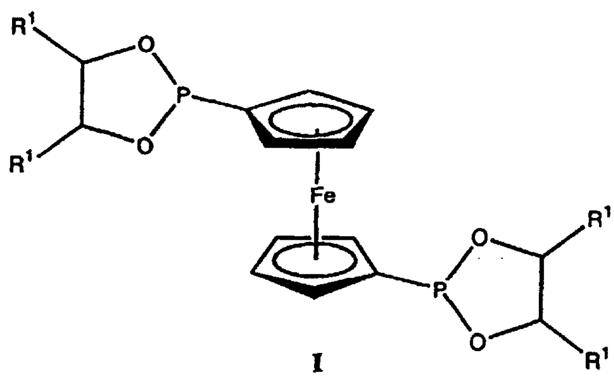

I

4. Diphosphonite nach Anspruch 3, wobei $R^1$ darstellt: Phenyl, 1-Naphthyl, 2-Naphthyl, Carboxypropyl, Carboxy-*iso*-propyl, Carboxybutyl, Carboxy-*tert*-butyl, Carboxyneopentyl oder Carboxyphenyl.

5. Diphosphonite nach Anspruch 1 und 2,
wobei deren Struktur der allgemeinen Formel **II** oder **III** entspricht,
wobei ein oder mehrere Kohlenstoffatome des Dinaphthol-Grundgerüsts in II durch Heteroatome ersetzt sein können,
wobei in Struktur III $R^4 \neq H$ ist,
wobei $R^1$, $R^2$, $R^3$, $R^5$ oder $R^6$ sowie in Struktur II $R^4$ Wasserstoff sein können oder $R^1$, $R^2$, $R^3$, $R^4$, $R^5$ und $R^6$ unabhängig voneinander nachfolgende Gruppen darstellen können:

gesättigte Kohlenwasserstoffe, die funktionalisiert und/oder verbrückend sein können, aromatische oder heteroaromatische Gruppen, die funktionalisiert und/oder anelliert sein können, nichtaromatische ungesättigte Kohlenwasserstoffe, die funktionalisiert sein können, Silylgruppen, Halogene (-Cl, -Br, -F oder -I), Nitro- ($-NO_2$) oder Nitrilgruppen (-CN), außerdem Ester ($-CO_2R$), Amide (-C(O)NRR'), Amine (-NRR'), Ether (-OR), Sulfide (-SR) oder Selenide (-SeR) in denen R und R' Substituenten repräsentieren, die Wasserstoff, gesättigte oder nichtaromatische ungesättigte Kohlenwasserstoffe, gegebenenfalls funktionalisiert, oder aromatische Reste, gegebenenfalls funktionalisiert, sind:

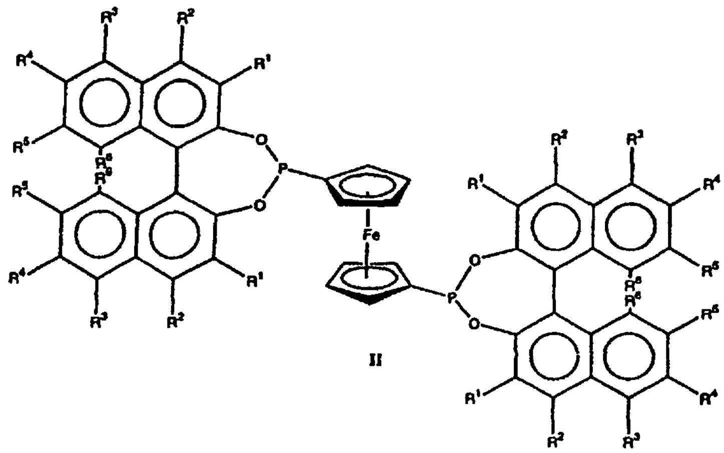

II

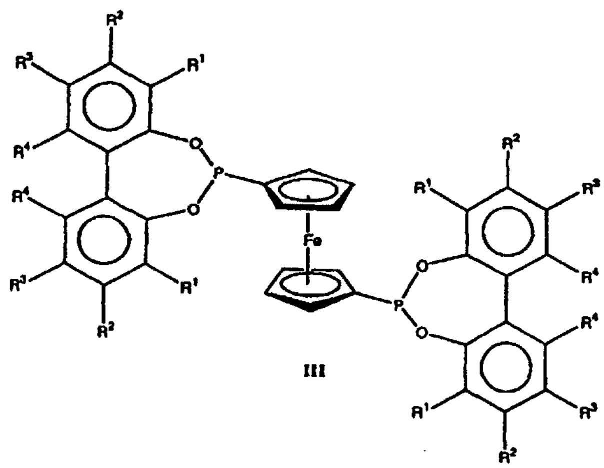

III

6. Diphosphonite nach Anspruch 5, wobei deren Struktur der allgemeinen Formel II entspricht, mit $R^1 = R^2 = R^3 = R^4 = R^5 = R^6 = H$.

7. Diphosphonite nach Anspruch 5, wobei deren Struktur der allgemeinen Formel **II** entspricht, wobei $R^2 = R^3 = R^4 = R^5 = R^6 = H$ und einem Rest $R^1$, der darstellen kann: Methyl, Ethyl, *n*-Propyl, *iso*-Propyl, *tert*-Butyl, Phenyl, 2,5-Dimethylphenyl, 2,5-Di-*tert*-butylphenyl, $-NO_2$, -Br, $-SiR_3$, -C≡C-R, $-C{\equiv}C-SiR_3$, $-CO_2R$ oder $-NR_2$, wobei R ein gesättigter Kohlenwasserstoff oder aromatischer Rest ist.

8. Diphosphonite nach Anspruch 5, wobei deren Struktur der allgemeinen Formel **III** entspricht, mit $R^1 = R^2 = H$ und $R^3 + R^4 = -(CH_2)_4-$.

9. Diphosphonite nach Anspruch 5, wobei deren Struktur der allgemeinen Formel **III** entspricht, wobei $R^2 = H$, $R^3 + R^4 = -(CH_2)_4-$ und $R^1$ darstellen kann: Methyl, Ethyl, *n*-Propyl, *iso*-Propyl, *tert*-Butyl, Phenyl, 2,5-Dimethylphenyl, 2,5-Di-*tert*-butylphenyl, $-NO_2$, $-SiR_3$, -C≡C-R, $-C{\equiv}C-SiR_3$, $-CO_2R$ oder $-NR_2$, wobei R ein gesättigter Kohlenwasserstoff oder aromatischer Rest ist.

10. Diphosphonite nach Anspruch 1 und 2, wobei deren Struktur der allgemeinen Formel **IV** entspricht, mit X = Stickstoff, Schwefel oder Sauerstoff und Resten $R^1$ (nur für X = N vorhanden), $R^2$, $R^3$, $R^4$ und $R^5$, wobei $R^4 \neq H$ und $R^1$, $R^2$, $R^3$ und $R^5$ Wasserstoff sein können oder $R^1$, $R^2$, $R^3$, $R^4$ und $R^5$ unabhängig voneinander nachfolgende Gruppen darstellen können: gesättigte Kohlenwasserstoffe, die funktionalisiert und/oder verbrückend sein können, aromatische oder heteroaromatische Gruppen, die funktionalisiert und/oder anelliert sein können, nichtaromatische ungesättigte Kohlenwasserstoffe, die funktionalisiert sein können, Silylgruppen, Halogene (-Cl, -Br, -F oder -I), Nitro- ($-NO_2$) oder Nitrilgruppen (-CN), außerdem Ester ($-CO_2R$), Amide (-C(O)NRR'), Amine (-NRR'), Ether (-OR), Sulfide (-SR) oder Selenide (-SeR), in denen R und R' Substituenten repräsentieren, die Wasserstoff, gesättigte oder nichtaromatische ungesättigte Kohlenwasserstoffe, gegebenenfalls funktionalisiert, oder aromatische Reste, gegebenenfalls funktionalisiert, sind:

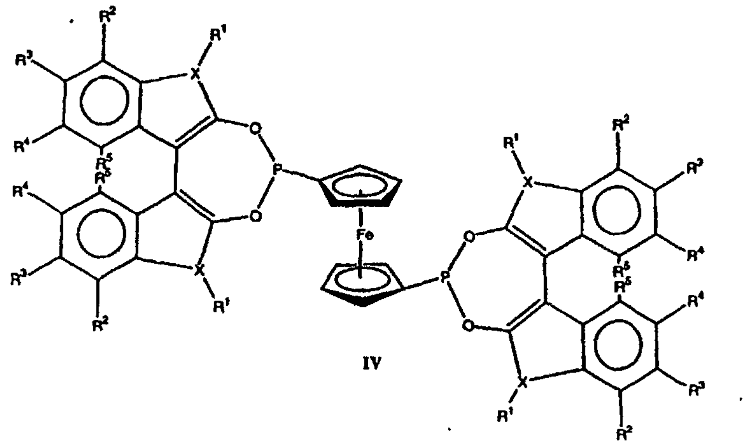

IV

**11.** Verfahren zur Herstellung eines chiralen $C_2$-symmetrischen Diphosphonits mit Ferrocen als Rückgrat gemäß Anspruch 1 bis 10, wobei das Verfahren einen Syntheseschritt enthält, in dem ein 1,1'-Ferrocenylendiphosphonigsäuretetraamid, dessen Struktur der allgemeinen Formel **IX** entspricht, mit einem entsprechenden Diol zum Diphosphonit umgesetzt wird:

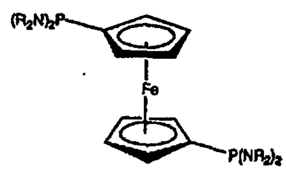

IX

**12.** Verfahren zur Herstellung eines chiralen $C_2$-symmetrischen Diphosphonits mit Ferrocen als Rückgrat gemäß Anspruch 1 bis 10, wobei das Verfahren einen Syntheseschritt enthält, in dem 1,1'-Bis(dichlorphosphino)ferrocen ohne Zusatz weiterer Reagentien oder Katalysatoren mit einem entsprechenden Diol zum Diphosphonit umgesetzt wird.

**13.** Chirale Übergangsmetall-Komplexe bestehend aus einem Diphosphonit gemäß Anspruch 1 bis 10 und einem Übergangsmetall der Gruppen VIIb, VIII oder Ib des Periodensystems.

**14.** Übergangsmetallkomplexe gemäß Anspruch 13 der allgemeinen Formel **XI** bis **XXXIV,** wobei Tf $=SO_2CF_3$ ist und in denen X folgende Anionen darstellen kann: Tetrafluoroborat, Hexafluorophosphat, Hexafluoroantimonat(V) oder $BAr_4^-$, wobei Ar Phenyl oder 2,5-Bis(trifluormethyl)phenyl ist (in den Formeln **XI** bis **XXXVIII** steht cod für $\eta^2$;$\eta^2$-1,5-Cyclooctadien, cymol für $\eta^6$-1-*iso*-Propyl-4-methylbenzol):

[**I**·Rh(cod)X] **(XI)**

[**II**·Rh(cod)X] **(XII)**

[**III**·Rh(cod)X] **(XIII)**

[**IV**·Rh(cod)X] **(XIV)**

[**I**·Ru(cymol)Cl]X **(XV)**

[**II**·Ru(cymol)Cl]X **(XVI)**

[**III**·Ru(cymol)Cl]X **(XVII)**

[**IV**·Ru(cymol)Cl]X **(XVIII)**

[**I**·Ir(cod)X] **(XIX)**

[**II**·Ir(cod)X] **(XX)**

[**III**·Ir(cod)X] **(XXI)**

[**IV**·Ir(cod)X] **(XXII)**

[**I**·Ni(cod)] **(XXIII)**

[**II**·Ni(cod)] **(XXIV)**

[**III**·Ni(cod)] **(XXV)**

[**IV**·Ni(cod)] **(XXVI)**

[**I**·Pd($CH_3$)$_2$] **(XXVII)**

[**II**·Pd($CH_3$)$_2$] **(XXVIII)**

[**III**·Pd($CH_3$)$_2$] **(XXIX)**

[**IV**·Pd($CH_3$)$_2$] **(XXX)**

[**I**·CuOTf] **(XXXI)**

[**II**·CuOTf] **(XXXII)**

[**III**·CuOTf] **(XXXIII)**

[**IV**·CuOTf] **(XXXIV)**

[**I**·Cu(OTf)$_2$] **(XXXV)**

[**II**· Cu(OTf)$_2$] **(XXXVI)**

[**III**· Cu(OTf)$_2$] **(XXXVII)**

[**IV**· Cu(OTf)$_2$] **(XXXVIII)**

**15.** Verwendung von chiraten Übergangsmetall-Komplexen gemäß Anspruch 13 oder 14 zur enantioselektiven Hydrierung, Hydroformylierung, Hydrocyanierung, Hydrosilylilierung oder Hydrovinylierung von Olefinen, Ketonen oder Iminen.

**16.** Verwendung von chiralen Übergangsmetall-Komplexen gemäß Anspruch 13 oder 14 zur enantioselektiven Hydrierung von Olefinen, Ketonen oder Iminen.

**17.** Verwendung nach Anspruch 15, wobei als Übergangsmetallverbindungen solche der allgemeinen Formel **XI** bis **XXII** verwendet werden, in denen X folgende Anionen darstellen kann: Tetrafluoroborat, Hexafluorophosphat, Hexafluoroantimonat(V) oder $BAr_4^-$, wobei Ar Phenyl oder 2,5-Bis(trifluormethyl)phenyl ist (in-den Formeln **XI** bis **XXII** steht cod für $\eta^2$;$\eta^2$-1,5-Cyclo- octadien und cymol für $\eta^6$-1-*iso*-Propyl-4-methylbenzol).

**18.** Verwendung von chiralen Übergangsmetall-Komplexen nach Anspruch 13 zur enantioselektiven Hydroborierung von Olefinen, wobei als Übergangsmetallverbindungen solche der allgemeinen Formel **XI** bis **XIV** und **XIX** bis **XXII** verwendet werden.

**19.** Verwendung von chiralen Übergangsmetall-Komplexen nach Anspruch 13 zur enantioselektiven 1,4-Addition an aktivierte Olefine (z.B. $\alpha,\beta$-ungesättigte Ketone), wobei als Übergangsmetallverbindungen solche der allgemeinen Formel **XXXI** bis **XXXVIII** verwendet werden.

**20.** Verwendung nach Anspruch 19, wobei als aktivierte Olefine $\alpha,\beta$-ungesättigte Ketone eingesetzt werden.

## Claims

**1.** Chiral $C_2$-symmetric diphosphonites comprising ferrocene as a backbone, **characterized by** containing either chiral $C_2$-symmetric 1,2-diols with an aliphatic basic structure or axially chiral aromatic or heteroaromatic diols in the P/O heterocycle.

**2.** The diphosphonites according to claim 1, being optically pure and pure with respect to diastereomers.

**3.** The diphosphonites according to claims 1 and 2, their structure corresponding to general formula I

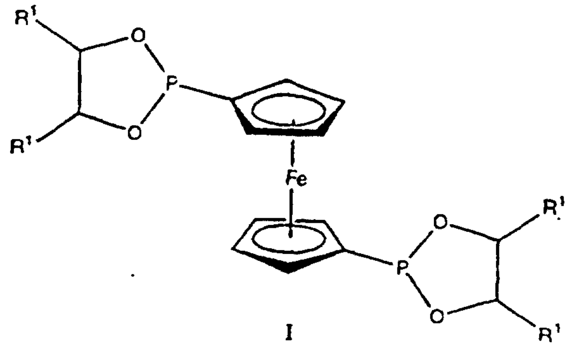

I

wherein $R^1$ is a saturated hydrocarbon which may optionally be functionalized, a non-aromatic unsaturated hydrocarbon which may optionally be functionalized, an aromatic or heteroaromatic group which may optionally be functionalized, an ester ($-CO_2R$) or an amide (-C(O)NRR'), wherein R and R' represent substituents which are hydrogen, saturated or non-aromatic unsaturated hydrocarbons which may optionally be functionalized, or aromatic residues which may optionally be functionalized.

**4.** The diphosphonites according to claim 3, wherein $R^1$ represents phenyl, 1-naphthyl, 2-naphthyl, carboxypropyl, carboxy-iso-propyl, carboxybutyl, carboxy-tert-butyl, carboxyneopentyl or carboxyphenyl.

**5.** The diphosphonites according to claims 1 and 2, wherein their structures correspond to general formula II or III; wherein one or more carbon atoms of the binaphthol core in II may be replaced by heteroatoms;
wherein $R^4 \neq H$ in structure III;
wherein $R^1$, $R^2$, $R^3$, $R^5$ or $R^6$ as well as, in structure II, $R^4$ can be hydrogen, or $R^1$, $R^2$, $R^3$, $R^4$, $R^5$ and $R^6$ may independently represent the following groups: saturated hydrocarbons which may be functionalized and/or bridging, aromatic or heteroaromatic groups which may be functionalized and/or condensed, non-aromatic unsaturated hydrocarbons which may be functionalized, silyl groups, halogens (-Cl, -Br, -F, -I), nitro ($-NO_2$) or nitrile (-CN) groups, or esters ($-CO_2R$), amides (-C(O)NRR'), amines (-NRR'), ethers (-OR), sulfides (-SR) or selenides (-SeR), wherein R and R' represent substituents which are hydrogen, saturated or non-aromatic unsaturated hydrocarbons, optionally functionalized, or aromatic residues, optionally functionalized:

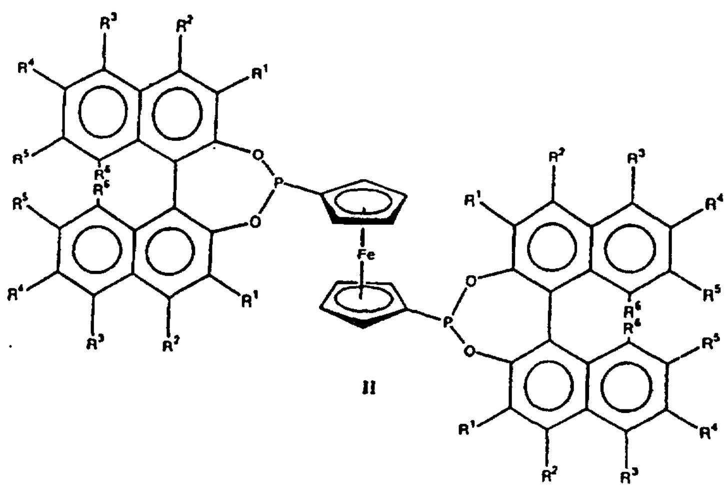

II

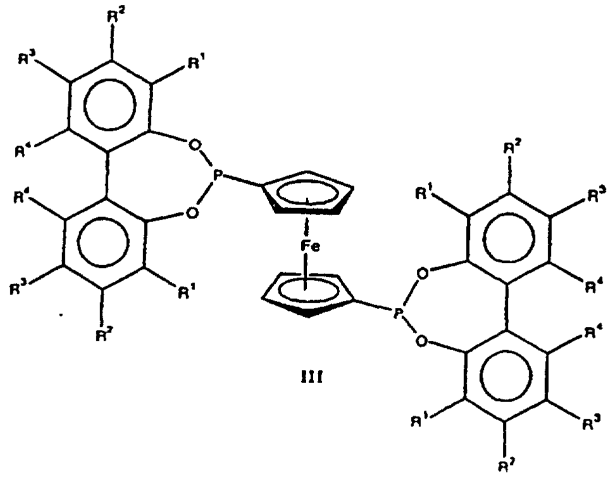

III

6. The diphosphonites according to claim 5, their structures corresponding to general formula II with $R^1 = R^2 = R^3 = R^4 = R^5 = R^6 = H$.

7. The diphosphonites according to claim 5, their structures corresponding to general formula II with $R^2 = R^3 = R^4 = R^5 = R^6 = H$ and a residue $R^1$ which may represent methyl, ethyl, n-propyl, iso-propyl, tert-butyl, phenyl, 2,5-dimethylphenyl, 2,5-di-tert-butylphenyl, $-NO_2$, -Br, $-SiR_3$, -C≡C-R, $-C≡C-SiR_3$, $-CO_2R$ or $-NR_2$, wherein R is a saturated hydrocarbon or aromatic residue.

8. The diphosphonites according to claim 5, their structures corresponding to general formula III with $R^1 = R^2 = H$ and $R^3 + R^4 = -(CH_2)_4-$.

9. The diphosphonites according to claim 5, their structures corresponding to general formula III wherein $R^2 = H$, $R^3 + R^4 = -(CH_2)_4-$, and a residue $R^1$ which may represent methyl, ethyl, n-propyl, iso-propyl, tert-butyl, phenyl, 2,5-dimethylphenyl, 2,5-di-tert-butylphenyl, $-NO_2$, $-SiR_3$, -C≡C-R, $-C≡C-SiR_3$, $-CO_2R$ or $-NR_2$, wherein R is a saturated hydrocarbon or aromatic residue.

10. The diphosphonites according to claims 1 and 2, their structures corresponding to general formula IV

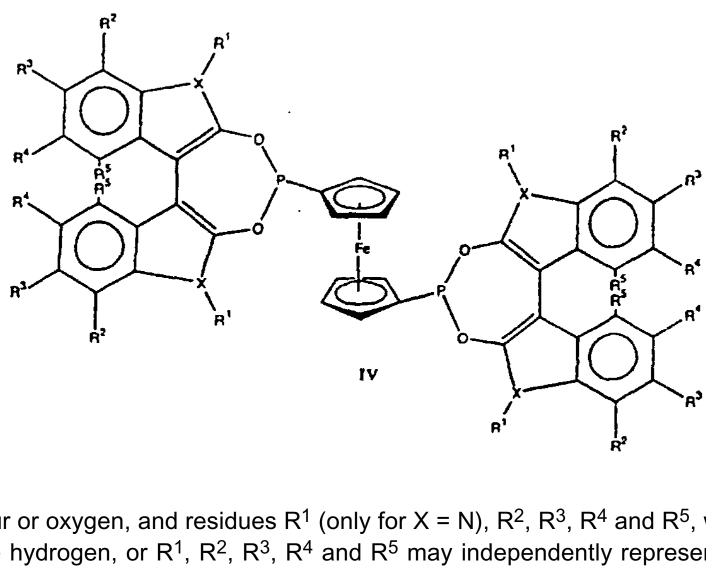

IV

with X = nitrogen, sulfur or oxygen, and residues $R^1$ (only for X = N), $R^2$, $R^3$, $R^4$ and $R^5$, wherein $R^4 \neq H$ and $R^1$, $R^2$, $R^3$ and $R^5$ may be hydrogen, or $R^1$, $R^2$, $R^3$, $R^4$ and $R^5$ may independently represent the following groups: saturated hydrocarbons which may optionally be functionalized and/or bridging, aromatic or heteroaromatic groups which may optionally be functionalized and/or condensed, non-aromatic unsaturated hydrocarbons which may optionally be functionalized, silyl groups, halogens (-Cl, -Br, -F or -I), nitro ($-NO_2$) or nitrile (-CN) groups, or esters

(-$CO_2$R), amides (-C(O)NRR'), amines (-NRR'), ethers (-OR), sulfides (-SR) or selenides (-SeR), wherein R and R' represent substituents selected from hydrogen, saturated or non-aromatic unsaturated hydrocarbons which may optionally be functionalized, or aromatic residues which may optionally be functionalized.

**11.** A process for the preparation of a chiral $C_2$-symmetric diphosphonite comprising ferrocene as a backbone according to claims 1 to 10, said process comprising a synthetic step in which a 1,1'-ferrocenylenediphosphonous acid tetraamide with a structure corresponding to general formula IX

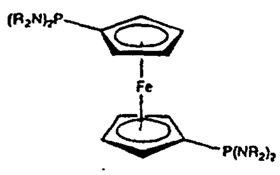

IX

is reacted with an appropriate diol to form the diphosphonite.

**12.** A process for the preparation of a chiral $C_2$-symmetric diphosphonite comprising ferrocene as a backbone according to claims 1 to 10, said process comprising a synthetic step in which a 1,1'-bis(dichlorophosphino)ferrocene is reacted with an appropriate diol to form the diphosphonite without adding further reagents or catalysts.

**13.** Chiral transition metal complexes consisting of a diphosphonite according to claims 1 to 10 and a transition metal of Periodic Table groups VIIb, VIII or Ib.

**14.** The transition metal complexes according to claim 13 of general formula XI to XXXVIII

[**I**·Rh(cod)X] **(XI)**

[**II**·Rh(cod)X] **(XII)**

[**III**·Rh(cod)X] **(XIII)**

[**IV**·Rh(cod)X] **(XIV)**

[**I**·Ru(cymol)Cl]X **(XV)**

[**II**·Ru(cymol)Cl]X **(XVI)**

[**III**·Ru(cymol)Cl]X **(XVII)**

[**IV**·Ru(cymol)Cl]X **(XVIII)**

[**I**·Ir(cod)X] **(XIX)**

[**II**·Ir(cod)X] **(XX)**

[**III**·Ir(cod)X] **(XXI)**

[**IV**·Ir(cod)X] **(XXII)**

[**I**·Ni(cod)] **(XXIII)**

[**II**·Ni(cod)] **(XXIV)**

[**III**·Ni(cod)] **(XXV)**

[**IV**·Ni(cod)] **(XXVI)**

[**I**·Pd$(CH_3)_2$] **(XXVII)**

[**II**·Pd$(CH_3)_2$] **(XXVIII)**

[**III**·Pd$(CH_3)_2$] **(XXIX)**

[**IV**·Pd$(CH_3)_2$] **(XXX)**

[**I**·CuOTf] **(XXXI)**

[**II**·CuOTf] **(XXXII)**

[**III**·CuOTf] **(XXXIII)**

[**IV**·CuOTf] **(XXXIV)**

[**I**·Cu$(OTf)_2$] **(XXXV)**

[**II**· Cu$(OTf)_2$] **(XXXVI)**

[**III**· Cu$(OTf)_2$] **(XXXVII)**

[**IV**· Cu$(OTf)_2$] **(XXXVIII)**

wherein Tf = $SO_2CF_3$ and X may represent the following anions: tetrafluoroborate, hexafluorophosphate, hexafluoroantimonate (V) or $BAr_4^-$,
wherein Ar is phenyl or 2,5-bis(trifluoromethyl)phenyl, cod standing for $\eta^2$:$\eta^2$-1,5-cyclooctadiene and cymol standing for $\eta^6$-1-iso-propyl-4-methylbenzene in formulae XI to XXXVIII.

**15.** Use of chiral transition metal complexes according to claim 13 or 14 for the enantioselective hydrogenation, hydroformylation, hydrocyanation, hydrosilylation or hydrovinylation of olefins, ketones or imines.

**16.** Use of chiral transition metal complexes according to claim 13 or 14 for the enantioselective hydrogenation of olefins, ketones or imines.

**17.** The use according to claim 15, wherein the transition metal compounds employed are those of general formulae XI to XXII wherein X may represent the following anions: tetrafluoroborate, hexafluorophosphate, hexafluoroantimonate(V) or $BAr_4^-$, wherein Ar is phenyl or 2,5-bis(trifluoromethyl)phenyl, cod standing for $\eta^2$:$\eta^2$-1,5-cyclooctadiene and cymol standing for $\eta^6$-1-isopropyl-4-methylbenzene in formulae XI to XXII.

**18.** Use of chiral transition metal complexes according to claim 13 for the enantioselective hydroboration of olefins, wherein the transition metal compounds employed are those of general formulae XI to XIV and XIX to XXII.

**19.** Use of chiral transition metal complexes according to claim 13 for the enantioselective 1,4-addition to activated olefins (e.g., $\alpha,\beta$-unsaturated ketones), wherein the transition metal compounds employed are those of general formulae XXXI to XXXVIII.

**20.** The use according to claim 19, wherein $\alpha,\beta$-unsaturated ketones are employed as said activated olefins.

**Revendications**

**1.** Diphosphonites chiraux symétriques en $C_2$ avec du ferrocène comme ossature, **caractérisés en ce qu'**ils contiennent dans l'hétérocycle P/O soit des 1,2-diols à symétrie chirale en $C_2$ avec ossature aliphatique, soit des diols aromatiques ou hétéroaromatiques à chiralité axiale.

**2.** Diphosphonites selon la revendication 1, qui sont énantiomériquement et diastéréomériquement purs.

**3.** Diphosphonites selon les revendications 1 et 2, dont la structure correspond à la formule générale I, dans laquelle $R^1$ représente un hydrocarbure saturé, éventuellement fonctionnalisé, un hydrocarbure insaturé non aromatique, éventuellement fonctionnalisé, un groupe aromatique ou hétéroaromatique éventuellement fonctionnalisé, un ester ($-CO_2R$) ou un amide (-C(O)NRR'), où R et R' représentent des substituants qui sont l'hydrogène, des hydrocarbures saturés ou insaturés non aromatiques, éventuellement fonctionnalisés, ou des radicaux aromatiques, éventuellement fonctionnalisés :

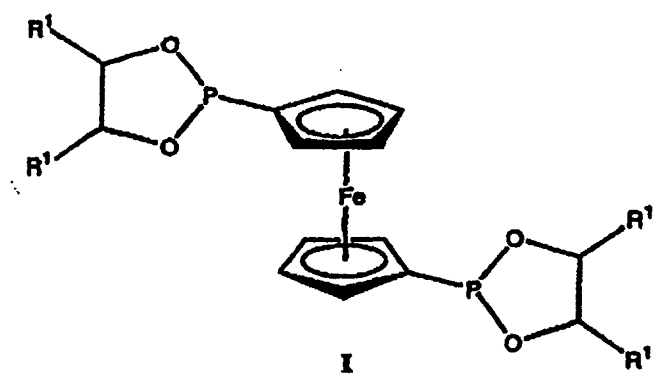

I

**4.** Diphosphonites selon la revendication 3, dans lesquels $R^1$ représente un phényle, un 1-naphtyle, un 2-naphtyle, un carboxypropyle, un carboxy-*iso*-propyle, un carboxybutyle, un carboxy-*tert*-butyle, un carboxynéopentyle ou un carboxyphényle.

**5.** Diphosphonites selon les revendications 1 et 2,
dont la structure correspond aux formules générales II ou III,
où un ou plusieurs des atomes de carbone de l'ossature de base dinaphtol dans II peuvent être remplacés par

des hétéroatomes,
où dans la structure III $R^4$ est différent de H,
où $R^1$, $R^2$, $R^3$, $R^5$ ou $R^6$ ainsi que dans la structure II $R^4$ peuvent représenter l'hydrogène, ou $R^1$, $R^2$, $R^3$, $R^4$, $R^5$ et $R^6$ peuvent représenter indépendamment l'un de l'autre les groupes suivants :

des hydrocarbures saturés, qui peuvent être fonctionnalisés et/ou faire partie d'un pont,
des groupes aromatiques ou hétéroaromatiques, qui peuvent être fonctionnalisés et/ou condensés, des hydrocarbures insaturés non aromatiques, qui peuvent être fonctionnalisés, des groupes silyle, des halogènes (-Cl, -Br, -F ou -I), des groupes nitro ($-NO_2$) ou des groupes nitrile (-CN), ainsi que des esters ($-CO_2R$), des amides (-C(O)NRR'), des amines (-NRR'), des éthers (-OR), des sulfures (-SR) ou des séléniures (-SeR) dans lesquels R et R' représentent des substituants qui sont l'hydrogène, des hydrocarbures saturés ou non aromatiques insaturés, éventuellement fonctionnalisés, ou des radicaux aromatiques, éventuellement fonctionnalisés:

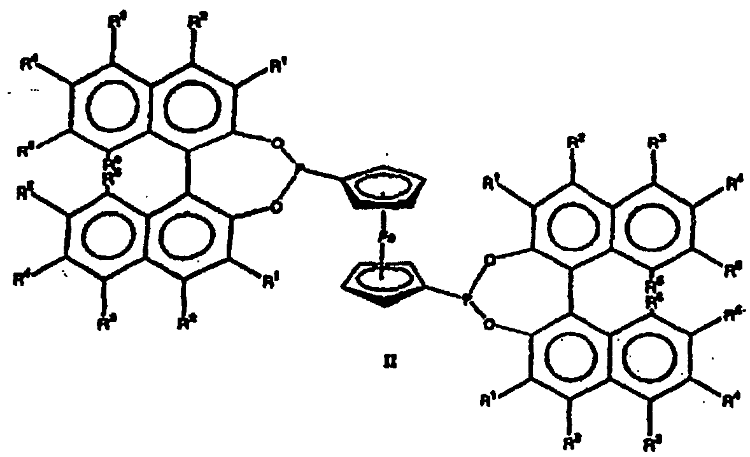
II

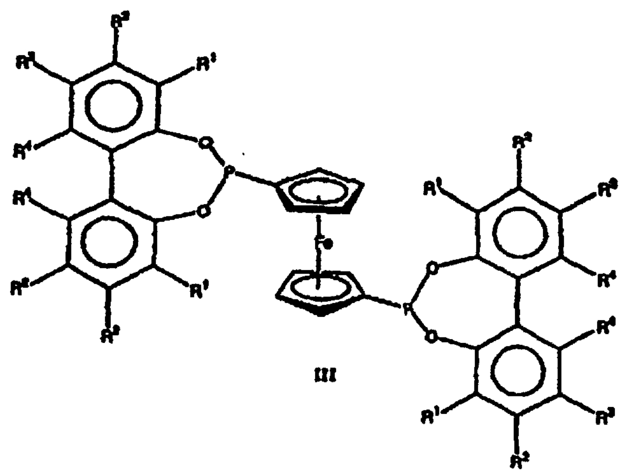
III

**6.** Diphosphonites selon la revendication 5, dont la structure correspond à la formule générale II, avec $R^1 = R^2 = R^3 = R^4 = R^5 = R^6 = H$.

**7.** Diphosphonites selon la revendication 5, dont la structure correspond à la formule générale II, dans laquelle $R^2 = R^3 = R^4 = R^5 = R^6 = H$ et un radical $R^1$ qui peut représenter : méthyle, éthyle, *n*-propyle, *iso*-propyle, *tert*-butyle, phényle, 2,5-diméthylphényle, 2,5-di-*tert*-butylphényle, $-NO_2$, -Br, $-SiR_3$, -C≡C-R, $-C{\equiv}C-SiR_3$, $-CO_2R$ ou $-NR_2$, où R est un hydrocarbure saturé ou un radical aromatique.

**8.** Diphosphonites selon la revendication 5, dont la structure correspond à la formule générale III, avec $R^1 = R^2 = H$ et $R^3 + R^4 = -(CH_2)_4-$.

**9.** Diphosphonites selon la revendication 5, dont la structure correspond à la formule générale III, dans laquelle $R^2$ = H, $R^3 + R^4 = -(CH_2)_4-$ et $R^1$ peut représenter : méthyle, éthyle, *n*-propyle, *iso*-propyle, *tert-butyle,* phényle, 2,5-diméthylphényle, 2,5-di-*tert*-butylphényle, $-NO_2$, $-SiR_3$, $-C\equiv C-R$, $-C\equiv C-SiR_3$, $-CO_2R$ ou $-NR_2$, où R est un hydrocarbure saturé ou un radical aromatique.

**10.** Diphosphonites selon la revendication 1 et 2, dont la structure correspond à la formule générale IV, avec X = azote, soufre ou oxygène, et les radicaux $R^1$ (uniquement lorsque X = N), $R^2$, $R^3$, $R^4$ et $R^5$, où $R^4 \neq H$ et $R^1$, $R^2$, $R^3$, $R^4$ et $R^5$ peuvent être un hydrogène, ou $R^1$, $R^2$, $R^3$, $R^4$ et $R^5$ peuvent représenter indépendamment l'un de l'autre les groupes suivants :

des hydrocarbures saturés, qui peuvent être fonctionnalisés et/ou faire partie d'un pont, des groupes aromatiques ou hétéroaromatiques, qui peuvent être fonctionnalisés et/ou condensés, des hydrocarbures insaturés non aromatiques, qui peuvent être fonctionnalisés, des groupes silyle, des halogènes (-Cl, -Br, -F ou -I), des groupes nitro ($-NO_2$) ou des groupes nitrile (-CN), ainsi que des esters ($CO_2R$), des amides (-C(O)NRR'), des amines (-NRR'), des éthers (-OR), des sulfures (-SR), ou des séléniures (-SeR), où R et R' représentent des substituants qui sont l'hydrogène, des hydrocarbures saturés ou non aromatiques insaturés, éventuellement fonctionnalisés, ou des radicaux aromatiques, éventuellement fonctionnalisés :

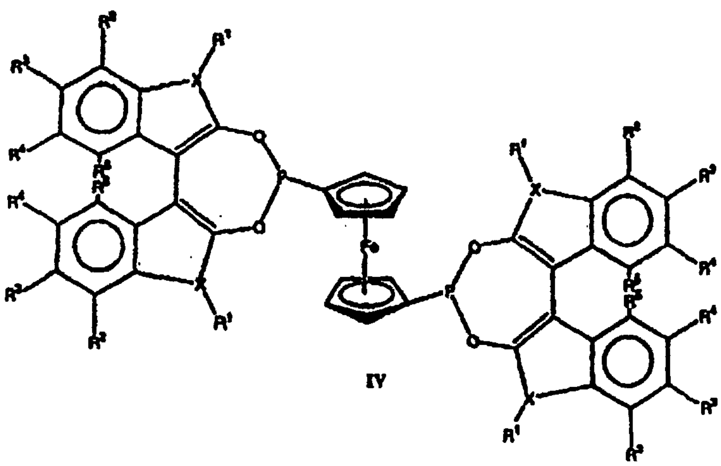

**11.** Procédé de préparation d'un diphosphonite à symétrie chirale en $C_2$ avec le ferrocène comme ossature selon les revendications 1 à 10, le procédé comprenant une étape de synthèse dans laquelle on fait réagir un tétraamide de l'acide 1,1'-ferrocénylène-diphosphonique dont la structure correspond à la formule générale IX, avec un diol correspondant pour donner le diphosphonite :

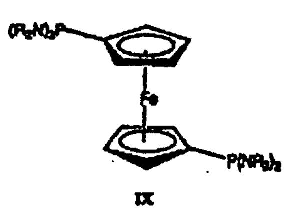

**12.** Procédé de préparation d'un diphosphonite chiral à symétrie en $C_2$, avec le ferrocène comme ossature selon l'une des revendications 1 à 10, le procédé incluant une étape de synthèse dans laquelle on fait réagir le 1,1'-bis-(dichlorophosphino)ferrocène sans addition d'autres réactifs ou catalyseurs avec un diol correspondant pour donner le diphosphonite.

**13.** Complexes chiraux de métaux de transition constitués d'un diphosphonite selon l'une des revendications 1 à 10 et d'un métal de transition des groupes VIIb, VIII ou Ib de la classification périodique des éléments.

**14.** Complexes de métaux de transition selon la revendication 13 de formules générales XI à XXXIV, où Tf = $SO_2CF_3$ et où X peut représenter les anions suivants : tétrafluoroborate, hexafluorophosphate, hexafluoroantimonate (V) ou $BAr_4^-$, où Ar représente un phényle ou un 2,5-bis(trifluorométhyl)phényle (dans les formules XI à XXXVIII, cod représente $\eta^2;\eta^2$-1,5-cyclooctadiène, cymol le $\eta^6$-1-*iso*-propyl-4-méthylbenzène) ;

[I·Rh(cod)X] (XI)

[II·Rh(cod)X] (XII)

[III·Rh(cod)X] (XIII)

[IV·Rh(cod)X] (XIV)

[I·Ru(cymol)Cl]X (XV)

[II·Ru(cymol)Cl]X (XVI)

[III·Ru(cymol)Cl]X (XVII)

[IV·Ru(cymol)Cl]X (XVIII)

[I·Ir(cod)X] (XIX)

[II·Ir(cod)X] (XX)

[III·Ir(cod)X] (XXI)

[IV·Ir(cod)X] (XXII)

[I·Ni(cod)] (XXIII)

[II·Ni(cod)] (XXIV)

[III·Ni(cod)] (XXV)

[IV·Ni(cod)] (XXVI)

[I·Pd($CH_3$)$_2$] (XXVII)

[II·Pd($CH_3$)$_2$] (XXVIII)

$[III \cdot Pd(CH_3)_2]$ (XXIX)

$[IV \cdot Pd(CH_3)_2]$ (XXX)

[I·CuOTf] (XXXI)

[II·CuOTf] (XXXII)

[III·CuOTf] (XXXIII)

[IV·CuOTf] (XXXIV)

$[I \cdot Cu(OTf)_2]$ (XXXV)

$[II \cdot Cu(OTf)_2]$ (XXXVI)

$[III \cdot Cu(OTf)_2]$ (XXXVII)

$[IV \cdot Cu(OTf)_2]$ (XXXVIII)

**15.** Utilisation de complexes de métaux de transition chiraux selon la revendication 13 ou 14 pour l'hydrogénation, l'hydroformylation, l'hydrocyanation, l'hydrosylilation, ou l'hydrovinylation énantiosélective d'oléfines, de cétones ou d'imines.

**16.** Utilisation de complexes de métaux de transition chiraux selon les revendications 13 ou 14 pour l'hydrogénation énantiosélective d'oléfines, de cétones ou d'imines.

**17.** Utilisation selon la revendication 15, dans laquelle on utilise comme composés de métaux de transition ceux de formules générales XI à XXII dans lesquelles X peut représenter les anions suivants: tétrafluoroborate, hexafluorophosphate, hexafluoroantimonate (V) ou $BAr_4^-$, où Ar représente un phényle ou un 2,5-bis(trifluorométhyl)phényle (dans les formules XI à XXII cod représente le $\eta^2;\eta^2$-1,5-cyclooctadiène, cymol le $\eta^6$-1-*iso*-propyl-4-méthylbenzène).

**18.** Utilisation de complexes de métaux de transition chiraux selon la revendication 13 pour l'hydroboration énantiosélective d'oléfines, où on utilise comme composés de métaux de transition ceux de formules générales XI à XIV et XIX à XXII.

**19.** Utilisation de complexes chiraux de métaux de transition selon la revendication 13 pour la 1,4-addition énantiosélective sur des oléfines actives (par exemple des cétones $\alpha,\beta$-insaturées), où on utilise comme composés de métaux de transition ceux de formules générales XXXI à XXXVIII.

**20.** Utilisation selon la revendication 19, où l'on utilise comme oléfines activées des cétones $\alpha,\beta$-insaturées.